# EUROPÄISCHE PATENTSCHRIFT

(11) **EP 3 272 301 B1**
(45) Veröffentlichungstag und Bekanntmachung des Hinweises auf die Patenterteilung: **01.05.2019**
(21) Anmeldenummer: 17178449.9
(22) Anmeldetag: 28.06.2017
(51) Int. Cl.: A61B 17/88, B01F 13/04, B01F 15/00, B05C 17/005, B05C 17/01

(54) **KNOCHENZEMENT-APPLIKATOR MIT DREIWEGE-VENTIL ZUR DRUCKENTLASTUNG**
BONE CEMENT APPLICATOR WITH THREE-WAY VALVE FOR PRESSURE RELIEF
APPLICATEUR DE CIMENT OSSEUX À L'AIDE D'UNE VANNE TROIS VOIES, DESTINÉ À LA DÉCHARGE DE PRESSION

(30) Priorität: 21.07.2016 DE 102016113468
(43) Veröffentlichungstag der Anmeldung: 24.01.2018
(73) Patentinhaber: Heraeus Medical GmbH, 61273 Wehrheim (DE)
(72) Erfinder: Vogt, Sebastian Dr., 99092 Erfurt (DE); Kluge, Thomas Dr., 56179 Vallendar (DE)
(74) Vertreter: Schultheiss & Sterzel Patentanwälte PartG mbB

(56) Entgegenhaltungen:
- WO-A1-2007/000066
- DE-A1-102011 010 763
- US-A1- 2005 105 384
- US-A1- 2010 262 152

## Beschreibung

Die Erfindung betrifft einen Knochenzement-Applikator zur Applikation eines Knochenzementteigs im Bereich der Wirbelsäule, der also insbesondere für die Vertebroplastie geeignet ist.

Die Erfindung betrifft auch ein Verfahren zur Applikation eines Knochenzementteigs, insbesondere eines pastenförmigen Mehrkomponenten-Polymethylmethacrylat-Knochenzementteigs, mit einem solchen Knochenzement-Applikator.

Gegenstand der Erfindung ist insbesondere ein einfacher, kostengünstig herzustellender Knochenzement-Applikator für die Vertebroplastie mit pastenförmigen Mehrkomponenten-Polymethylmethacrylat-Knochenzementen, mit dem Ausgangskomponenten des Polymethylmethacrylat-Knochenzementteigs mit manuell bedienbaren Auspressvorrichtungen gemischt und ausgetragen werden können oder mit dem der vorgemischte Polymethylmethacrylat-Knochenzementteig selbst mit manuell bedienbaren Auspressvorrichtungen ausgetragen werden kann.

Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Diese Polymethylmethacrylat-Knochenzemente werden nach Vermischung des Zementpulvers mit der flüssigen Monomerkomponente im noch nicht ausgehärteten, pastenförmigen Zustand als Knochenzementteig appliziert. Bei Verwendung von Mischsystemen befindet sich der Knochenzementteig im Fall von Pulver-Flüssigkeits-Zementen in einer Kartusche. Aus dieser Kartusche wird der Knochenzementteig durch Bewegung eines Austragskolbens heraus gedrückt. Die Austragskolben haben üblicherweise einen Durchmesser zwischen 30 mm bis 40 mm und damit an der Außenseite, an welcher der Stößel der Auspressvorrichtung beim Auspressvorgang angreift, eine Fläche von 7,0 cm² bis 12,5 cm². Die Bewegung des Austragskolbens wird durch manuell bedienbare, mechanische Auspressvorrichtungen bewirkt, die auch als Applikatoren bezeichnet werden. Diese manuellen Auspressvorrichtungen erreichen normalerweise eine Auspresskraft im Bereich von ungefähr 1,5 kN bis 3,5 N.

Eine neuere Entwicklung stellen pastenförmige Zweikomponenten-Knochenzemente dar, wie sie beispielsweise aus der DE 10 2007 050 762 B3, der DE 10 2008 030 312 A1 und der DE 10 2007 052 116 B4 bekannt sind. Bei diesen Zweikomponenten-Knochenzementen werden zwei pastenförmige Ausgangskomponenten in zwei separaten Kartuschen mit zwei separaten Austragskolben aufbewahrt. Bei der Applikation werden beide Pasten durch Bewegung der Austragkolben aus den Kartuschen in einen statischen Mischer gepresst und nach erfolgter Vermischung durch ein Austragsrohr ausgetragen. Bei geeigneter Zusammensetzung der pastenförmigen Ausgangskomponenten wird nach der Vermischung der beiden Ausgangskomponenten sofort ein klebfreier, applikationsbereiter Zementteig erhalten. Wartezeiten bis zum Erreichen der Klebfreiheit des Zementteigs, die bei bisherigen konventionellen Polymethylmethacrylat-Knochenzementen immer zwangsläufig auftreten, entfallen dadurch. Wertvolle OP-Zeit kann dadurch gespart werden.

Bei der Applikation der bisherigen, konventionellen PMMA-Knochenzemente, die aus einer flüssigen Monomerkomponente und einer separat dazu gelagerten Zementpulverkomponente als Ausgangskomponenten bestehen, wird nach der Vermischung der beiden Ausgangskomponenten in Zementiersystemen beziehungsweise Vakuumzementiersystemen der gebildete Zementteig mit Hilfe von manuell bedienbaren Auspressvorrichtungen ausgepresst. Diese einfachen mechanischen Auspressvorrichtungen nutzen insbesondere Klemmstangen als Stößel zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei hochviskosen pastenförmigen Ausgangskomponenten unter Verwendung von Kartuschen, bei denen die Austragskolben an den äußeren Kolbenseiten, an denen die Stößel der Auspressvorrichtungen angreifen, eine gesamte Fläche im Bereich von 7,0 cm² bis 12,5 cm² haben, sind diese Vorrichtungen nicht oder nur mit einem sehr großen Kraftaufwand manuell zu bedienen. Dieser große Kraftaufwand ist medizinischen Anwendern im OP nicht zuzumuten.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A; DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1; US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

Die US 8,544,683 B2 offenbart ein Kartuschensystem, das zur Beimischen einer geringen Menge zu einer Hauptausgangskomponente geeignet ist. Bei dem Kartuschensystem ist neben einer Kartusche eine zweite kleinere Kartusche angeordnet, wobei bei einem Vortrieb eines Austragskolbens in der größeren Kartusche auch ein Austragskolben in der kleineren Kartusche über ein gemeinsames Verbindungselement angetrieben wird. Das System ist zum Mischen der zähen pastösen Ausgangskomponenten von PMMA-Knochenzement jedoch nicht geeignet.

Ein koaxiales Kartuschensystem, das ein spezielles Kolbensystem enthält, wird im Patent EP1 392 450 B1 beschrieben. Das Kartuschensystem findet Anwendung in der Baustoffchemie für die Lagerung und Vermischung von pastenförmigen Zweikomponentendichtungsmassen.

Im Patent FR 1 468 507 wird ein Kartuschensystem offenbart, bei dem in einer Kartusche ein schlauchförmiger Lagerbehälter angeordnet ist. Dieser ist am Kartuschenende an einem Punkt mit der Kartusche verbunden. Bei der Vorwärtsbewegung des Austragskolbens in Richtung Kartuschenkopf wird einerseits die in der Kartusche enthaltene Masse ausgepresst und andererseits wird aus dem schlauchförmigen Behälter die darin enthaltene Masse durch Ausquetschen in Richtung Kartuschenkopf bewegt.

In der Vertebroplastie wird die Applikation von Knochenzementteig mit einem Röntgenverfahren in situ verfolgt. Bei Applikationsvorrichtungen für die Vertebroplastie wird üblicherweise ein Schlauch eingesetzt, über dessen Spitze der Knochenzementteig appliziert werden kann, damit der Anwender außerhalb des Bereichs der Röntgenstrahlung arbeiten kann. Hierzu können am Schlauch des Weiteren ein Trokar oder eine Kanüle angeordnet sein. Derartige Systeme sind beispielsweise aus US 7 112 205 B2, US 8 038 682 B2, US 8 308 731 B2, DE 10 2005 045 227 A1, EP 1 074 231 B1, EP 1 596 736 B1, US 9 005 209 B2 und WO 2008/097855 A2 bekannt.

Alternativ können auch andere Aufbauten verwendet werden, um den Anwender von der Röntgenstrahlung fernzuhalten, wie beispielsweise in den Dokumenten US 6 676 663 B2, US 7 008 433 B2, US 8 348 494 B2, EP1 464 292 B1, EP 1 614403 B1, US 2008/319445 A9 und WO 2008/038322 A2 beschrieben.

Die WO 2007/000066 A1 offenbart ein Ventilelement, bei dem eine Paste, die mit einem statischen Mischer gemischt wurde, bei geschlossenem Ventil von dem Mischer in eine Kammer geleitet wird. Aus der US 2010/0262152 A1 ist ein System zum Mischen von Knochenzementpulver mit einer Monomerflüssigkeit bekannt, bei dem über ein Ventil ein Vakuum in einem Mischraum erzeugt werden kann, um die Monomerflüssigkeit in das Zementpulver zu saugen.

Ein Knochenzement-Applikator für die Vertebroplastie zum Applizieren von Knochenzementteig mit einem Schlauch, einem Trokar und einem Mischer ist aus der US 2008/0086143 A1 bekannt. Der Knochenzement-Applikator umfasst zwei nebeneinander angeordnete Kartuschen, in denen die Ausgangskomponenten auch gelagert werden. Der Knochenzement-Applikator wird unmittelbar vor der Anwendung zusammengesetzt. Bei derartigen Knochenzement-Applikatoren für die Vertebroplastie wird auf die Ausgangskomponenten des Knochenzementteigs mit Hilfe einer Auspressvorrichtung, die Austragskolben in den Kartuschen vortreiben, ein Druck ausgeübt und mit Hilfe des Drucks die Ausgangskomponenten aus den Kartuschen und durch den Schlauch ausgetrieben. Dabei werden die Ausgangskomponenten meist zuvor in einem vorgeschalteten statischen Mischer gemischt. Dies führt dazu, dass sich die den Knochenzementfluss begrenzenden Teile des Knochenzement-Applikators (die Kartuschen, das Gehäuse des Mischers und der Schlauch) elastisch verformen. Wenn der Vortrieb der Austragskolben gestoppt wird, kommt es durch die elastische Kraft dieser Teile zu einer Volumenkontraktion dieser Teile, so dass auch dann noch Knochenzementteig durch die Applikationsöffnung des Schlauchs beziehungsweise Trokars austritt. Hierdurch kann es zu einer Kontamination des OP-Saals oder des Anwenders mit dem Knochenzementteig kommen oder es wird ungewollt eine zu große Menge des Knochenzementteigs appliziert. Zudem muss, wenn der Volumenstrom des Knochenzementteigs wieder gestartet werden soll, zunächst wieder der Druck im Knochenzementteig aufgebaut werden, damit dieser durch die Applikationsöffnung austritt. Dadurch verzögert sich wiederum der Zeitpunkt nach dem Beginn des Vortreibens der Austragskolben, ab dem tatsächlich der Knochenzementteig appliziert werden kann, was ebenfalls unerwünscht ist.

Aufgrund der großen Zähigkeit des Knochenzementteigs und gegebenenfalls der Ausgangskomponenten sind alle diese Effekte bei solchen Knochenzement-Applikatoren relativ stark ausgeprägt. Dem kann durch die Verwendung massiver und teurer metallisches Gehäuseteile entgegengewirkt werden. Diese Teile müssen nach der Anwendung gereinigt und für eine weitere Anwendung sterilisiert werden oder sie müssen aufwendig recycelt werden.

Die Aufgabe der Erfindung besteht also darin, die Nachteile des Stands der Technik zu überwinden. Insbesondere soll ein einfacher und kostengünstig zu fertigender Knochenzement-Applikator für die Vertebroplastie für pastenförmige Mehrkomponenten-Polymethylmethacrylat-Knochenzemente und ein Verfahren zum Applizieren eines Knochenzementteigs mit einem Knochenzement-Applikator für die Vertebroplastie bereitgestellt werden, der einfach aufgebaut ist und kostengünstig zu fertigen ist, wobei es beim Stoppen des Knochenzementflusses nicht zu einem Nachlaufen des Knochenzementteigs kommen soll. Ferner soll der Knochenzement-Applikator nach Unterbrechung des Flusses des Knochenzementteigs möglichst schnell wieder einsetzbar sein. Es soll eine Kontamination der Umgebung und des Anwenders mit Knochenzementteig möglichst ausgeschlossen werden.

Der Erfindung liegt insbesondere die Aufgabe zu Grunde, einen einfachen Knochenzement-Applikator als Misch- und Applikationssystem für einen Pulver-Flüssigkeits-PMMA oder nur als Applikationssystem für die Vertebroplastie bereitzustellen. Der Knochenzement-Applikator soll möglichst einfach aus Kunststoff gefertigt werden können und sich dadurch als Produkt zur einmaligen Anwendung eignen. Das Auspressen des gemischten Knochenzementteigs soll mit einer konventionellen, manuell angetriebenen Auspressvorrichtung, wie sie bisher bei PMMA-Knochenzementen zur Zementierung von Knie- und Hüft-TEP üblich ist, möglich sein. Der Knochenzement-Applikator soll so beschaffen sein, dass ein sofortiger Notstop des fließenden Knochenzementteigs möglich ist, ohne dass eine Kontamination des OPs mit dem Knochenzementteig erfolgt.

Des Weiteren soll der Knochenzement-Applikator als einmalig zu verwendendes ready-to-use-System in einfachster Weise mit einer minimalen Anzahl von Montageschritten innerhalb weniger Sekunden einsatzbereit machbar sein und nach Verbindung mit manuell anzutreibenden medizinischen Auspressvorrichtungen sofort nach Beginn der manueller Betätigung der Auspressvorrichtung einen homogen gemischten Knochenzementteig erzeugen und an der Applikationsöffnung eines Schlauchs austragen, möglichst auch dann wenn der Fluss des Knochenzementteigs kurzzeitig unterbrochen wurde. Die in den OPs bisher für die konventionellen Polymethylmethacrylat-Knochenzemente genutzten manuell zu bedienenden Auspressvorrichtungen mit jeweils einer Schubstange beziehungsweise mit jeweils einem Stößel und gegebenenfalls einem Teller sollen für den Austrag des Zweikomponenten-Polymethylmethacrylat-Knochenzements beziehungsweise des Knochenzementteigs mit dem zu entwickelnden Knochenzement-Applikator genutzt werden können. Dadurch soll die Beschaffung von speziellen Auspressvorrichtungen für den Austrag von pastenförmigen Zweikomponenten-Polymethylmethacrylat-Knochenzementen vermieden werden.

Die Ausgangskomponenten des Knochenzementteigs sollen bevorzugt in dem Knochenzement-Applikator mit einander gemischt werden können. Alternativ kann auch ein bereits gemischter Knochenzementteig oder ein vorgemischter Knochenzementteig verwendet werden.

Der Knochenzement-Applikator soll bevorzugt auch ein geringes Volumen des homogen gemischten Knochenzementteigs von ca. 50 ml beziehungsweise maximal 80 ml austragen können, ohne dass größere Restmengen (mehr als 20 ml) in dem System verbleiben und aufwendig entsorgt werden müssen. Größere Volumina des Zementteigs werden für Anwendungen in der Vertebroplastie üblicherweise nicht angestrebt.

Der Knochenzement-Applikator soll so beschaffen sein, dass eine Verwechselung der relevanten Montageschritte durch den Anwender konstruktiv soweit wie möglich ausgeschlossen ist und der Knochenzement-Applikator auch von weitgehend ungeschultem Personal anwendbar ist. Weiterhin soll ein Verfahren zum Austragen eines Knochenzementteigs bereitgestellt werden, das bevorzugt auch zum Vermischen der Ausgangskomponenten vorgesehen ist.

Die Aufgaben der Erfindung werden gelöst durch einen Knochenzement-Applikator zur Applikation eines Knochenzementteigs im Bereich der Wirbelsäule, der Knochenzement-Applikator aufweisend
eine röhrenförmige Kartusche mit einem Innenraum,
einen Austragskolben zum Austreiben der Ausgangskomponenten des Knochenzementteigs oder des Knochenzementteigs aus der Kartusche durch eine dem Austragskolben gegenüberliegende Öffnung der Kartusche, wobei der Austragskolben in Längsrichtung im Innenraum der Kartusche beweglich ist,
einen Schlauch,
eine Applikationsöffnung, durch die der Knochenzementteig zu applizieren ist,
ein von außen bedienbares Dreiwege-Ventil, das im Schlauch oder an einer der Kartusche zugewandten Seite des Schlauchs angeordnet oder anzuordnen ist, wobei das Dreiwege-Ventil mit der Öffnung der Kartusche in Fluidverbindung steht, wenn das Dreiwege-Ventil mit der Kartusche verbunden ist,
einen Auffangbehälter zum Aufnehmen von Knochenzementteig, der an dem Dreiwege-Ventil angeordnet ist,
wobei das Dreiwege-Ventil derart aufgebaut und in dem Knochenzement-Applikator angeordnet oder anzuordnen ist, dass es
in einer ersten Stellung eine Fluidverbindung zwischen der Applikationsöffnung und der Öffnung der Kartusche bereitstellt und eine Durchführung zum Auffangbehälter verschließt und in einer zweiten Stellung eine Fluidverbindung zwischen der Applikationsöffnung und dem Auffangbehälter bereitstellt und einen Durchgang zur Öffnung der Kartusche verschließt.

Der Knochenzement-Applikator ist erfindungsgemäß bevorzugt für die Vertebroplastie vorgesehen und damit für die Vertebroplastie einsetzbar und geeignet.

Das Dreiwege-Ventil ist erfindungsgemäß bevorzugt manuell bedienbar.

Bevorzugt ist das Dreiwege-Ventil fest mit dem Schlauch verbunden.

Besonders bevorzugt liegen die Kartusche sowie das Dreiwege-Ventil mit dem Schlauch oder das Dreiwege-Ventil und der Schlauch als einzelne miteinander verbindbare Teile vor.

Es kann vorgesehen sein, dass der Schlauch die Applikationsöffnung aufweist, insbesondere einen Luer-System-Adapter oder einen Trokar mit der Applikationsöffnung aufweist, und/oder eine der Applikationsöffnung gegenüberliegenden Anschlussöffnung aufweist.

Die Applikationsöffnung ist in Fluidverbindung mit dem Schlauch.

Auf der Seite der Kartusche bedeutet, dass die Anordnung bezogen auf die Flussrichtung des Knochenzementteigs beziehungsweise der Ausgangskomponenten bei geeigneter Stellung des Dreiwege-Ventils erfolgt, das heißt, auf der Seite erfolgt, von der aus der Knochenzementteig beziehungsweise die Ausgangskomponenten einfließt, wenn das Dreiwege-Ventil mit der Kartusche verbunden ist.

In dem Schlauch oder an dem der Applikationsöffnung gegenüberliegendem Ende des Schlauchs ist das Dreiwege-Ventil angeordnet.

Das Dreiwege-Ventil oder das der Applikationsöffnung gegenüberliegende Ende des Schlauchs ist in Fluidverbindung mit der Öffnung der Kartusche, wenn der Schlauch und das Dreiwege-Ventil mit der Kartusche verbunden sind.

Es kann bevorzugt vorgesehen sein, dass zwischen der Kartusche und dem Dreiwege-Ventil oder zwischen der Kartusche und dem der Applikationsöffnung gegenüberliegende Ende des Schlauchs ein Rohr oder ein Rohr mit einem Mischer angeordnet oder anzuordnen ist.

Der Austragskolben der Kartusche ist vorzugsweise in dem der Öffnung der Kartusche gegenüberliegenden Ende der Kartusche angeordnet.

Es kann erfindungsgemäß vorgesehen sein, dass in Flussrichtung hinter der Öffnung der Kartusche oder zwischen der Öffnung der Kartusche und dem Dreiwege-Ventil oder dem Schlauch ein Mischer zum Mischen des Knochenzementteigs, insbesondere ein statischer Mischer, angeordnet oder anzuordnen ist, wobei vorzugsweise das Dreiwege-Ventil zwischen dem Mischer und dem Schlauch angeordnet oder anzuordnen ist, so dass das Dreiwege-Ventil in der ersten Stellung eine Fluidverbindung zwischen der Applikationsöffnung und dem Mischer bereitstellt und in der zweiten Stellung den Durchgang zum Mischer verschließt.

Hiermit wird erreicht, dass die Ausgangskomponenten oder der vorgemischte Knochenzementteig aus der Kartusche besser durchmischt werden. Durch die erfindungsgemäße Anordnung des Dreiwege-Ventils wird ferner erreicht, dass der Druck des Knochenzementteigs im Mischer erhalten bleibt und somit unmittelbar nach einem erneuten Öffnen des Dreiwege-Ventils wieder Knochenzementteig durch den Schlauch und die Applikationsöffnung ausgetrieben werden kann, ohne dass der Druck im Mischer neu aufgebaut werden müsste. Dadurch kann also eine schnellere Bereitstellung von Knochenzementteig nach dem Öffnen des Dreiwege-Ventils erreicht werden.

Mit der vorliegenden Erfindung wird auch vorgeschlagen, dass der Knochenzement-Applikator mit Hilfe einer manuell bedienbaren Auspressvorrichtung bedienbar ist und der Austragskolben mit manueller Kraft in der Kartusche bewegbar ist, wobei der Querschnitt des Innenraums der Kartusche maximal 3,5 cm² beträgt, bevorzugt maximal 2,5 cm² beträgt.

Dass der Austragskolben mit manueller Kraft in der Kartusche bewegbar ist, bedeutet, dass der Austragskolben mit einer manuell angetriebenen Auspressvorrichtung in der Kartusche bewegbar ist.

Durch diese maximalen Querschnitts- beziehungsweise Antriebsflächen, die die zum Austreiben und Mischen der Ausgangskomponenten des Knochenzementteigs oder die zum Austreiben des Knochenzementteigs selbst notwendige Kraft begrenzen, kann erreicht werden, dass der zähe Knochenzementteig aus der Kartusche mit manueller Kraft durch den Schlauch und gegebenenfalls den Mischer ausgetrieben werden kann. Dadurch kann ein manuell angetriebener beziehungsweise antreibbarer Knochenzement-Applikator bereitgestellt werden, der ohne einen Anschluss für eine externe Energiequelle und auch ohne eine interne Energiequelle auskommt und somit immer und unabhängig von den äußeren Gegebenheiten Einsatzbereit ist, beziehungsweise es kann eine Auspressvorrichtung verwendet werden, die ohne einen Anschluss für eine externe Energiequelle und auch ohne eine interne Energiequelle auskommt und somit immer und unabhängig von den äußeren Gegebenheiten Einsatzbereit ist.

Des Weiteren kann vorgesehen sein, dass der Schlauch zumindest bereichsweise flexibel ist und/oder die Applikationsöffnung in einem Anschluss mit einem Innengewinde, insbesondere in einem Luer-System-Adapter, oder in einem Trokar angeordnet ist.

Hiermit wird erreicht, dass der Knochenzement-Applikator auch in schwer zugänglichen Bereichen einsetzbar ist. Ferner wird über den Schlauch und gegebenenfalls den Trokar erreicht, dass der Eintrag des Knochenzementteigs mit einem Röntgenverfahren überwacht werden kann, ohne dass der Bediener des Knochenzement-Applikators der Röntgenstrahlung unmittelbar ausgesetzt ist. Der Trokar kann über ein Innengewinde, insbesondere über ein Luer-System, mit dem restlichen Schlauch verbunden sein. Mit Hilfe eines Luer-System-Adapters wird eine universelle Anschließbarkeit des Knochenzement-Applikators beziehungsweise von dessen Bauteilen erreicht.

Bevorzugt kann auch vorgesehen sein, dass der Auffangbehälter nach außen für den Knochenzementteig undurchlässig ist, vorzugsweise der Auffangbehälter flüssigkeitsdicht oder flüssigkeitsdicht und gasdicht ist, und/oder der Auffangbehälter ein Volumen aufweist, das mindestens so groß ist wie die Hälfte des Volumens des Schlauchs, bevorzugt mindestens so groß ist wie das Volumen des Schlauchs.

Hiermit kann vermieden werden, dass der Knochenzementteig und dessen Bestandteile nach außen gedrückt werden und dadurch den OP-Saal oder den Anwender verschmutzen beziehungsweise kontaminieren.

Ferner kann vorgesehen sein, dass die Kartusche an der Rückseite ein Befestigungselement zur Befestigung einer Auspressvorrichtung aufweist.

Hiermit wird eine stabile Befestigung der Auspressvorrichtung mit dem Knochenzement-Applikator ermöglicht.

Bevorzugte Ausführungsformen der vorliegenden Erfindung können sich dadurch auszeichnen, dass die Kartusche, der Austragskolben, das Dreiwege-Ventil und der Schlauch und gegebenenfalls der Mischer aus Kunststoff gefertigt sind, wobei als Kunststoffe Polyethylen-co-vinylalkohol (EVOH), Polybutylenterephthalat (PBT), Polyethylenterephthalat (PET) und Poly-methacrylsäuremethylester-co-acrylnitril bevorzugt werden.

Der Aufbau mit Kunststoffen ist kostengünstig und einfach umsetzbar. Die bevorzugten Kunststoffe sind aufgrund ihrer Resistenz gegenüber den in den Ausgangskomponenten und in dem Knochenzementteig enthaltenen Chemikalien besonders gut geeignet.
die Kartusche einen zylindrischen Innenraum aufweist und/oder der Austragskolben fluiddicht mit den Innenwänden der Kartusche abschließt, bevorzugt über zumindest eine umlaufende Dichtung und/oder eine Abstreiflippe dicht mit der Innenwand der Kartusche abschließt.

Gemäß einer bevorzugt Weiterbildung der vorliegenden Erfindung kann vorgesehen sein, dass die Öffnung der Kartusche in einem Kartuschenkopf angeordnet ist, insbesondere in einem mit der Kartusche lösbar verbundenen Kartuschenkopf angeordnet ist, wobei im Bereich der Öffnung ein Befestigungsmittel, insbesondere ein Außengewinde, vorgesehen ist, mit dem der Schlauch oder das Dreiwege-Ventil an der Kartusche zu befestigen ist oder befestigt ist, insbesondere über ein Dichtmittel druckdicht zu befestigen oder befestigt ist.

Hierdurch kann der Schlauch oder das Dreiwege-Ventil oder ein Rohr, das mit dem Dreiwege-Ventil oder dem Schlauch verbunden ist, leicht mit der Kartusche unmittelbar vor deren Einsatz verbunden werden.

Es kann erfindungsgemäße auch vorgesehen sein, dass eine Mischeinrichtung vorgesehen ist, mit der sich der Inhalt der Kartusche im Innenraum der Kartusche durchmischen lässt, wobei bevorzugt die Mischeinrichtung, wenn sie mit der Kartusche verbunden ist, von außen bedienbar ist.

Hierdurch können die Ausgangskomponenten in der Kartusche zu dem Knochenzementteig gemischt oder vorgemischt werden. Es besteht dann auch die erfindungsgemäß besonders bevorzugte Möglichkeit, den Knochenzementteig in einem Vakuum (beziehungsweise unter Unterdruck) zu mischen, indem der Innenraum der Kartusche über einen Vakuumanschluss evakuiert wird. Hierzu ist bevorzugt eine Porenscheibe in der Verbindung des Vakuumanschlusses zum Innenraum der Kartusche vorgesehen, wobei die Porenscheibe für Zementpulver undurchlässig ist.

Bei Knochenzement-Applikatoren mit einer Mischeinrichtung kann auch vorgesehen sein, dass mehrere mit einem Mischstab verbundene Mischflügel im Innenraum der Kartusche angeordnet sind, die durch eine Bewegung des Mischstabs, insbesondere durch axiale lineare Bewegung und durch Drehung des Mischstabs, im Innenraum der Kartusche zur Durchmischung von Ausgangskomponenten im Innenraum der Kartusche bewegbar sind, wenn die Mischeinrichtung mit der Kartusche verbunden ist.

Hierdurch kann der Knochenzementteig besonders einfach manuell gemischt werden. Zudem kann an der Konsistenz des Knochenzementteigs beim Mischen dessen Zustand erkannt werden.

Ferner kann vorgesehen sein, dass die Mischeinrichtung, insbesondere der Mischstab der Mischeinrichtung, durch die Öffnung der Kartusche in den Innenraum der Kartusche geführt ist, wobei die Mischeinrichtung aus der Kartusche entfernbar ist, bevorzugt aus der Kartusche herausziehbar ist, wenn sie mit der Kartusche verbunden ist.

Dann kann die Mischeinrichtung nach dem Mischen des Knochenzementteigs aus den Ausgangskomponenten vor dem Applizieren des Knochenzementteigs entfernt werden und bevorzugt durch den Schlauch und das Dreiwege-Ventil und gegebenenfalls zusätzlich durch den Trokar und einen Mischer ersetzt werden.

Es wird auch ein Verfahren zur Applikation eines Knochenzementteigs, insbesondere eines pastenförmigen Mehrkomponenten-Polymethylmethacrylat-Knochenzementteigs, mit einem erfindungsgemäßen Knochenzement-Applikator mit den folgenden nacheinander ablaufenden Schritten beschrieben:
a) Einfüllen eines Knochenzementteigs in den Innenraum der Kartusche oder Einfüllen der Ausgangskomponenten des Knochenzementteigs in den Innenraum der Kartusche,
b) Einsetzen des Knochenzement-Applikators in eine Auspressvorrichtung, die Auspressvorrichtung aufweisend einen axial vortreibbaren Stößel zum Vortreiben des Austragskolbens im Innenraum der Kartusche in Richtung der Öffnung der Kartusche,
c) das Dreiwege-Ventil wird in die erste Stellung gebracht oder es befindet sich in der ersten Stellung und es erfolgt ein Auspressen des Inhalts der Kartusche mit Hilfe der Auspressvorrichtung durch axiales Vortreiben eines Stößels der Auspressvorrichtung, wobei der Austragskolben vom Stößel in Richtung der Öffnung gedrückt wird, wodurch der Knochenzementteig durch den Schlauch und aus der Applikationsöffnung gedrückt wird oder wodurch die Ausgangskomponenten zum Knochenzementteig gemischt werden und der Knochenzementteig anschließend durch den Schlauch und aus der Applikationsöffnung gedrückt wird,
d) Überführen des Dreiwege-Ventils in die zweite Stellung,
e) durch die zweite Stellung des Dreiwege-Ventils wird der Fluss des Knochenzementteigs oder der Ausgangskomponenten aus der Kartusche in den Schlauch gestoppt und ein Teil des zwischen der Applikationsöffnung und dem Dreiwege-Ventil im Schlauch unter Druck stehenden Knochenzementteigs wird durch das Dreiwege-Ventil in den Auffangbehälter gedrückt.

Wenn das Dreiwege-Ventil nicht schon mit der Kartusche verbunden ist, wird das Dreiwege-Ventil mit dem Schlauch (und gegebenenfalls mit einem statischen Mischer und/oder einem Trokar) vor Schritt c) mit der Kartusche verbunden.

Dabei kann vorgesehen sein, dass nach Schritt e) in einem Schritt f) das Dreiwege-Ventil wieder in die erste Stellung überführt wird und dadurch der Knochenzementteig wieder aus der Kartusche durch das Dreiwege-Ventil zur Applikationsöffnung geleitet wird, wobei bevorzugt anschließend die Schritte d), e) und f) chronologisch einmal oder mehrmals wiederholt werden.

Während das Dreiwege-Ventil geschlossen ist, das heißt, sich in der zweiten Stellung befindet, wird zweckmäßigerweise der Vortrieb des Stößels und damit der Vortrieb des Austragskolbens gestoppt und der Vortrieb erst dann wieder aufgenommen, wenn das Dreiwege-Ventil sich in der geöffneten ersten Stellung befindet.

Ferner kann vorgesehen sein, dass die Auspressvorrichtung manuell, mit Druckluft oder mit einem Motor angetrieben wird, wobei durch die manuelle Kraft, die Druckluft oder den Motor der Stößel in Richtung der Öffnung der Kartusche vorgetrieben wird.

Manuell antreibbare Auspressvorrichtungen sind bevorzugt, da sie weder an eine Druckluftquelle oder eine Energiequelle angeschlossen werden müssen, noch eine solche enthalten müssen.

Ferner kann bei Anwendung der ersten Ausführung vorgesehen sein, dass der Stößel der Auspressvorrichtung auf die von der Öffnung der Kartusche abgewandte Seite des Austragskolbens drückt und der Austragskolben den Stößel angetrieben wird.

An dem in Richtung der Kartusche weisenden Ende des Stößels der Auspressvorrichtung kann ein Teller angeordnet sein, mit dem auf den Austragskolben gedrückt wird, um den Austragskolben in der Kartusche vorzutreiben.

Es kann auch vorgesehen sein, dass in Schritt a) die Ausgangskomponenten oder der Knochenzementteig in den Innenraum der Kartusche gefüllt werden und die Ausgangskomponenten oder der Knochenzementteig in Schritt c) aus der Kartusche gepresst werden, vorzugsweise die Ausgangskomponenten oder der vorgemischte Knochenzementteig durch einen statischen Mischer gepresst werden, der zwischen dem Schlauch oder dem Dreiwege-Ventil und der Öffnung angeordnet ist, wobei bevorzugt die Ausgangskomponenten im Mischer zu einem Knochenzementteig gemischt werden.

Ferner kann vorgesehen sein, dass in Schritt a) die Ausgangskomponenten in den Innenraum der Kartusche gefüllt werden und vor Schritt b) mit einer Mischeinrichtung im Innenraum der Kartusche durchmischt werden, wobei danach der Schlauch mit dem Dreiwege-Ventil an der Kartusche, insbesondere an der Öffnung der Kartusche, befestigt werden, wobei bevorzugt vor dem Anschließen des Schlauchs mit dem Dreiwege-Ventil die Mischeinrichtung aus der Kartusche entfernt wird.

Der Erfindung liegt die überraschende Erkenntnis zugrunde, dass durch Einstellen eines Dreiwege-Ventils, das mit einem Auffangbehälter, einem Schlauch und zumindest einer Kartusche verbunden ist, der Druck von dem Knochenzementteig im Schlauch und gegebenenfalls im Trokar genommen werden kann, ohne dass eine größere Menge des Knochenzementteigs aus der Applikationsöffnung nachfließt. Gleichzeitig kann auf diese Weise der Druck des Knochenzementteigs und gegebenenfalls der Ausgangskomponenten in der Kartusche bis zum Dreiwege-Ventil und insbesondere gegebenenfalls auch im Mischer (sofern vorhanden), aufrechterhalten werden. Dadurch ist die Zeit, die nach dem Öffnen des Dreiwege-Ventils (nach Überführung in die erste Stellung) bis zum erneuten Austritt des Knochenzementteigs aus der Applikationsöffnung an der Spitze des Schlauchs oder Trokars vergeht, sehr kurz. Die Spannung des Knochenzement-Applikators wird zwischen dem Dreiwege-Ventil und dem Austragskolben also gehalten, wenn das Dreiwege-Ventil geschlossen wird (in der zweiten Stellung des Dreiwege-Ventils), während eine schnelle Entspannung des Knochenzement-Applikators zwischen dem Dreiwege-Ventil und der Applikationsöffnung durch Abfließen des Knochenzementteigs durch das Dreiwege-Ventil in der zweiten Stellung erreicht wird. Damit der Knochenzementteig nicht die Umgebung oder den Anwender kontaminiert ist ein Auffangbehälter vorgesehen, der ein Abtropfen des durch das Dreiwege-Ventil austretenden Knochenzementteigs verhindert. Bevorzugt ist hierzu der Auffangbehälter geschlossen. Theoretisch kann es ausreichen, wenn der Knochenzementteig zurückgehalten wird. Der Auffangbehälter kann auch flexibel und/oder elastisch sein und sich bei der Aufnahme des aus dem Dreiwege-Ventil austretenden Knochenzementteigs ausdehnen.

Der besondere Vorteil des erfindungsgemäßen Knochenzement-Applikators besteht darin, dass mit konventionellen, manuell angetriebenen Auspressvorrichtungen, die für normale PMMA-Zemente üblich sind, der Zweikomponenten-Spinezement beziehungsweise der gemischte Zweikomponenten-Knochenzementteig für die Vertebroplastie über einen dünnen Schlauch in den Trokar ausgepresst werden kann. Die Augmentation von Wirbelkörpern erfolgt unter permanenter Röntgenkontrolle. Der Schlauch zwischen dem Trokar und dem Applikator ermöglicht es, dass der Arzt mit seinen Händen nicht im Bereich der Röntgenstrahlung arbeitet. Es sind dabei keine komplexen, teuren Hydraulik-Applikationsvorrichtungen notwendig. Weiterhin ist es vorteilhaft, dass der Knochenzement-Applikator ein Notablassventil in Form des Dreiwege-Ventils enthält, mit dem der Auspressvorgang sofort gestoppt werden kann, wenn der Zementteig in unerwünschte Bereiche des Wirbelkörpers zu fließen beginnt. Dieses Notablassventil druckentlastet das Applikationssystem, in dem der Nachdruck des Zementteigs aus dem statischen Mischer blockiert wird und gleichzeitig der vor dem Notablassventil befindliche Zementteig druckentlastet wird, in dem ein Kanal in den Auffangbehälter geöffnet wird, in den der Zementteig austreten kann bis der Druck im Schlauch beziehungsweise im Trokar abgebaut ist. Durch den Auffangbehälter kommt es nicht zu einer Kontamination des OPs beziehungsweise der Handschuhe des Arztes durch den beim Notablass austretenden Knochenzementteig.

Eigene Versuche zeigten, dass während des Auspressvorgangs der Kartusche auf Grund der hohen Viskosität der pastenförmigen Ausgangskomponenten ein sehr großer Druckabfall im Schlauch, aber insbesondere auch an einem statischen Mischer (sofern vorhanden) stattfindet.

Weiterhin basiert die Erfindung auf der Beobachtung, dass hochviskoser Zementteig aus zylinderförmigen Kartuschen durch einen statischen Mischer und durch den Schlauch mit handelsüblichen manuell anzutreibenden Auspressvorrichtungen in akzeptabler Zeit und mit akzeptablem, da manuell aufbringbarem Kraftaufwand ausgetragen werden kann, wenn der Austragskolben an seiner Stirnseite einen Durchmesser von maximal 35 mm hat. Mit dem erfindungsgemäßen Aufbau wird ein Knochenzement-Applikator bereitgestellt, der derartig kleine Durchmesser für die Anwendung hochviskoser Knochenzementteige realisieren kann.

Dabei kann die Kartusche dennoch ohne zu großen Aufwand mit den Ausgangskomponenten befüllt werden.

Ein beispielhafter Knochenzement-Applikator ist zusammengesetzt aus
1. einer röhrenförmigen Kartusche mit einem Innendurchmesser kleiner, gleich 20 mm (bevorzugt 15 mm)
2. einem Austragskolben,
3. einem manuell entfernbaren Kartuschenkopf mit darin axial beweglich angeordneten manuell von außen zu bedienenden Mischelement,
7. einem Dreiwege-Ventil (als Notablassventil) am Kartuschenkopf (beziehungsweise genauer anbringbar und anzuordnen am Kartuschenkopf), mit einer seitlichen Notauslassöffnung,
8. einem hohlen Reservoir (dem Auffangbehälter), das so um das Notablassventil (dem Dreiwege-Ventil) angeordnet ist, dass die Notablassöffnung mit dem Hohlraum des Reservoirs verbunden ist, und
9. einem Schlauch, der an einem Ende mit dem Notablassventil und am anderen Schlauchende mit einem Luer-System-Adapter verbunden ist.

Im Folgenden werden weitere Ausführungsbeispiele der Erfindung anhand von siebzehn schematisch dargestellten Figuren erläutert, ohne jedoch dabei die Erfindung zu beschränken. Dabei zeigt:
Figur 1: eine schematische perspektivische Ansicht eines beispielhaften erfindungsgemäßen Knochenzement-Applikators;
Figur 2: eine schematische perspektivische Ansicht eines vergrößerten Ausschnitts, in dem das Dreiwege-Ventil des Knochenzement-Applikators nach Figur 1 gezeigt ist;
Figur 3: zwei schematische perspektivische Querschnittansichten durch das Dreiwege-Ventil des Knochenzement-Applikators nach den Figuren 1 und 2 und zwar das Dreiwege-Ventil in geschlossener Position beziehungsweise Stellung (Figur 3 oben) und in geöffneter Position beziehungsweise Stellung (Figur 3 unten);
Figur 4: zwei schematische Querschnittansichten in Aufsicht durch das Dreiwege-Ventil des Knochenzement-Applikators nach den Figuren 1 und 2 und zwar das Dreiwege-Ventil in geöffneter Position beziehungsweise Stellung (Figur 4 oben) und in geschlossener Position beziehungsweise Stellung (Figur 4 unten);
Figur 5: eine schematische Querschnittansicht des geöffneten Dreiwege-Ventils nach den Figuren 2, 3 und 4 des Knochenzement-Applikators, wobei die Schnittebene senkrecht zu den Schnittebenen nach den Figuren 3 und 4 gewählt ist;
Figur 6: eine schematische Querschnittansicht des Luer-System-Adapters an der Spitze des Knochenzement-Applikators als Ausschnittvergrößerung;
Figur 7: eine schematische perspektivische Ansicht eines erfindungsgemäßen Knochenzement-Applikators im Ausgangzustand;
Figur 8: eine schematische Querschnittansicht in Längsrichtung des Knochenzement-Applikators nach Figur 7;
Figur 9: eine schematische perspektivische Ansicht eines erfindungsgemäßen Knochenzement-Applikators mit angesetztem Dreiwege-Ventil;
Figur 10: links eine schematische Querschnittansicht in Längsrichtung des Knochenzement-Applikators nach Figur 9 und rechts ein Ausschnitt mit einem senkrechten Querschnitt in Längsrichtung;
Figur 11: eine schematische perspektivische Ansicht eines erfindungsgemäßen Knochenzement-Applikators bereit zur Applikation;
Figur 12: links eine schematische Querschnittansicht in Längsrichtung des Knochenzement-Applikators nach Figur 11 und rechts ein Ausschnitt mit einem senkrechten Querschnitt in Längsrichtung;
Figur 13: eine schematische Querschnittansicht eines gefüllten erfindungsgemäßen Knochenzement-Applikators vor dem Mischen;
Figur 14: eine schematische Querschnittansicht des gefüllten erfindungsgemäßen Knochenzement-Applikators nach Figur 13 nach dem Mischen;
Figur 15: eine schematische Querschnittansicht des gefüllten erfindungsgemäßen Knochenzement-Applikators nach Figur 13 und 14, bereit zur Applikation und eingesetzt in eine Auspressvorrichtung;
Figur 16: eine schematische Querschnittansicht des Knochenzement-Applikators nach den Figuren 13 bis 15 während des Auspressens des Knochenzementteigs; und
Figur 17: eine schematische Querschnittansicht des Knochenzement-Applikators nach den Figuren 13 bis 16 nach Auspressen des Knochenzementteigs.

In den Figuren werden der Einfachheit halber bei gleichen Bauteilen die gleichen Bezugszeichen auch für unterschiedliche Ausführungsformen verwendet.

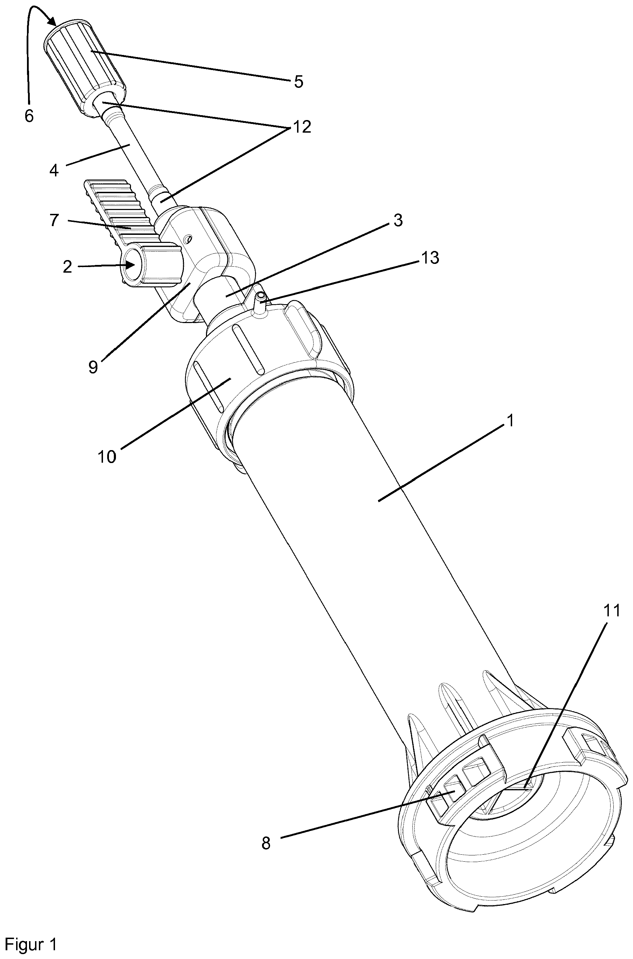

Die Figuren 1 bis 6 zeigen einen beispielhaften erfindungsgemäßen Knochenzement-Applikator für die Vertebroplastie. Figur 1 zeigt dabei eine schematische perspektivische Ansicht eines beispielhaften erfindungsgemäßen Knochenzement-Applikators. Der Knochenzement-Applikator umfasst eine Kartusche 1, ein Dreiwege-Ventil 2, ein Rohr 3 und einen Schlauch 4. Das Rohr 3 ist bevorzugt ein Ansatzstück des Dreiwege-Ventils 2, kann also als Teil des Dreiwege-Ventils 2 aufgefasst werden. In einen zylindrischen Innenraum (in Figur 1 nicht zu sehen) der Kartusche 1 können die Ausgangskomponenten des herzustellenden Knochenzementteigs oder ein fertig gemischter Knochenzementteig eingefüllt werden beziehungsweise sind in dem in Figur 1 gezeigten Aufbau bevorzugt bereits eingefüllt und vermischt worden. Zur Lagerung der Ausgangskomponenten ist der gezeigte Knochenzement-Applikator nicht vorgesehen und genaugenommen nicht geeignet. Die Kartusche 1 wurde kurz vor der Anwendung geöffnet und mit dem Dreiwege-Ventil 2, dem Rohr 3 und dem Schlauch 4 des Knochenzement-Applikators verbunden, nach dem der Knochenzementteig in die Kartusche 1 eingefüllt wurde oder nach die Ausgangskomponenten in der Kartusche 1 zum Knochenzementteig gemischt wurden.

An der Vorderseite des Schlauchs 4 ist ein Luer-System-Adapter 5 angeordnet, in dem sich eine Applikationsöffnung 6 befindet. An diesen Luer-System-Adapter 5 kann ein Trokar (in Figur 1 nicht gezeigt) mit einer Kanüle oder eine andere Verlängerung mit passendem Luer-System-Anschluss angeschlossen werden, mit dem der Knochenzementteig im Bereich der Wirbel appliziert werden kann. Der Trokar kann als Teil des Knochenzement-Applikators angesehen werden. Die Applikationsöffnung 6 wird durch den Trokar entsprechend verlängert, das heißt, dass die Applikationsöffnung 6 sich durch das Anschließen des Trokars an die Spitze des Trokars verlängert. Der Knochenzement-Applikator wird üblicherweise nicht ohne den Trokar betrieben und angewendet.

Das Dreiwege-Ventil 2 kann über einen Knebelgriff 7 manuell bedient werden, in dem es um 90° gedreht wird und damit von einer geschlossenen Position beziehungsweise von einer geschlossenen Stellung in eine geöffnete Position beziehungsweise in eine geöffnete Stellung überführt wird.

An der Rückseite der Kartusche 1 beziehungsweise am Kartuschenboden ist ein Anschluss 8 zum Anschließen einer Auspressvorrichtung (nicht gezeigt) angeordnet. Die Auspressvorrichtung wird an diesem Anschluss 8 befestigt, um den Inhalt der Kartusche 1, also den Knochenzementteig aus der Kartusche 1 auszupressen, dann in Flussrichtung durch das Rohr 3 hindurch, durch das (geöffnete) Dreiwege-Ventil 2 hindurch und durch den Schlauch 4 hindurch (und bevorzugt durch den Trokar hindurch) bis durch die Applikationsöffnung 6, über die der Knochenzementteig im Wirbel appliziert wird.

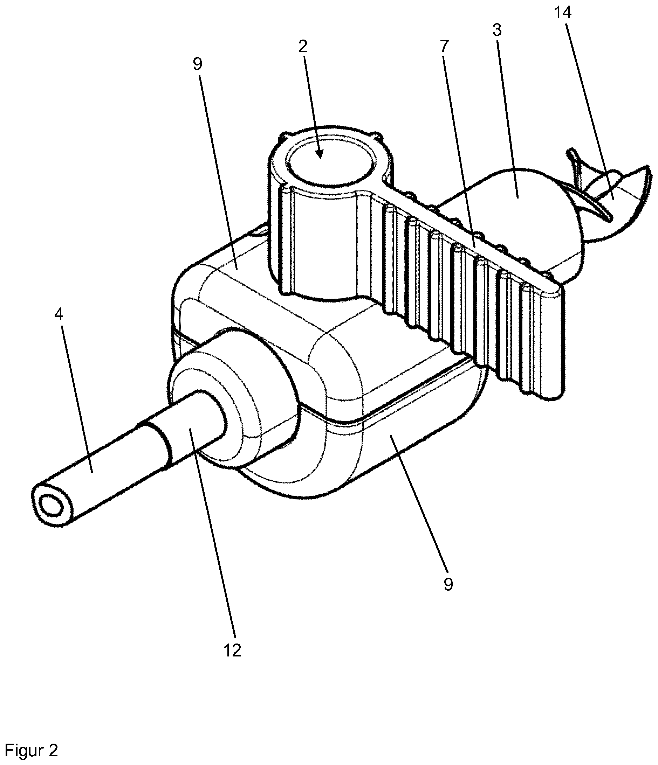

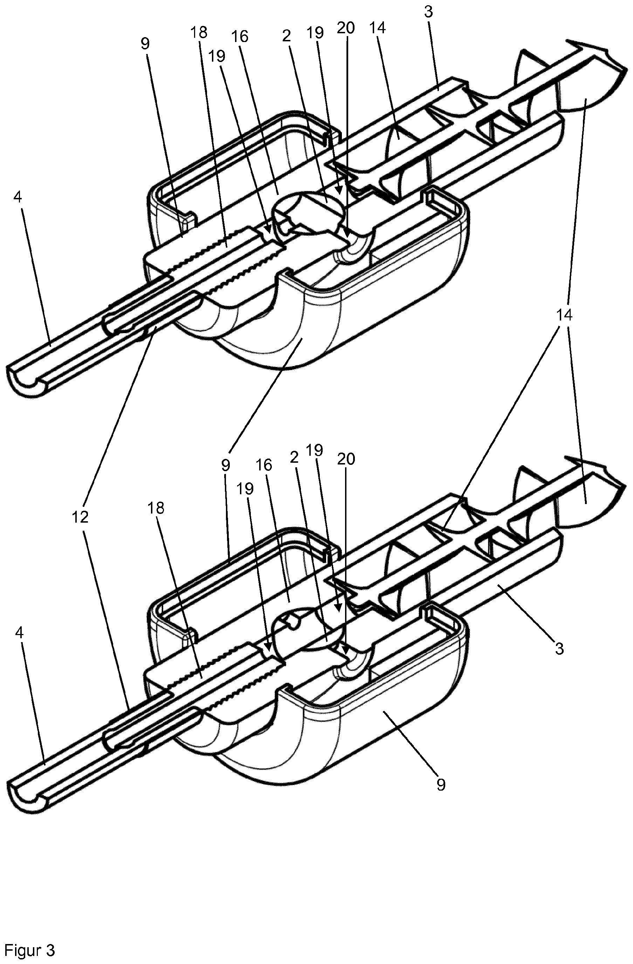

Im Bereich des Dreiwege-Ventils 2 ist ein Auffangbehälter 9 angeordnet, der das Dreiwege-Ventil 2 bezüglich der Längsachse des Knochenzement-Applikators axial umschließt. Der Auffangbehälter 9 ist aus zwei Kunststoffteilen zusammengesteckt (siehe Figuren 2 und 3).

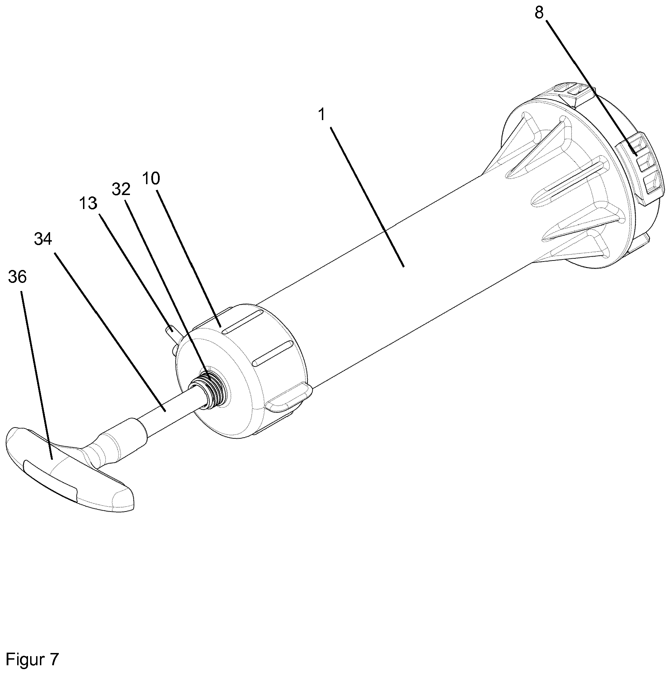

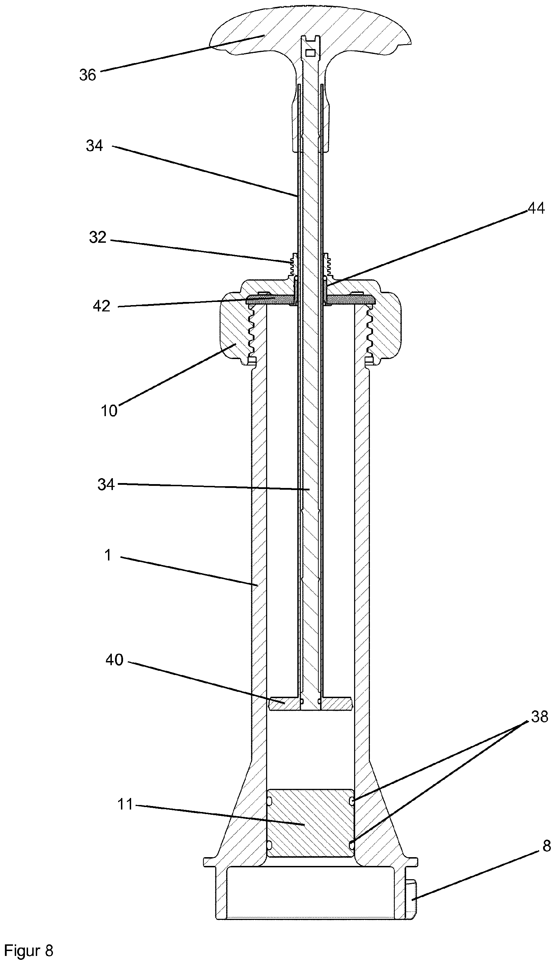

Die Kartusche 1 und das Rohr 3 sind an einer Überwurfmutter 10 miteinander verbunden. An der Kartusche 1 ist im Bereich des Kartuschenkopfs 10, der dem Kartuschenboden gegenüberliegt, ein zu einem Innengewinde der Überwurfmutter 10 passendes Außengewinde vorgesehen, auf das die Überwurfmutter 10 mit dem Innengewinde aufgeschraubt werden kann. Das Rohr 3 ist mit der Kartusche 1 verbunden, indem es auf einen Stutzen 32 (siehe Figuren 7, 8, 10 und 12 bis 17) an der Überwurfmutter 10 aufgeschraubt ist. Das Rohr 3 weist hierzu bodenseitig ein Innengewinde auf. Zwischen dem Rohr 3 und der Kartusche 1 ist vorzugsweise eine Dichtung vorgesehen. Bevor die Kartusche 1 mit dem Rohr 3 über die Überwurfmutter 10 verbunden ist, kann eine Mischeinrichtung (siehe Figuren 8, 9, 13 und 14) durch die Überwurfmutter 10 geführt sein, mit der der Inhalt der Kartusche 1 zu durchmischen ist.

Im Kartuschenboden ist ein Austragskolben 11 eingesetzt, mit dem der Inhalt der Kartusche 1 in das Rohr 3 aus der Kartusche 1 auszutreiben ist. Dazu ist der Austragskolben 11 in Längsrichtung im Innenraum der Kartusche 1 in Richtung der Überwurfmutter 10 zu drücken. Hierzu kann eine manuell angetriebene Auspressvorrichtung verwendet werden.

Das Dreiwege-Ventil 2 und der Luer-System-Adapter 5 sind über einen Crimpanschluss mit Hilfe von Hülsen 12 aus Metall mit dem Schlauch 4 druckdicht verbunden. Bis auf den Crimpanschluss können alle Teile des Knochenzement-Applikators aus Kunststoff gefertigt werden, wobei alle Dichtungen bevorzugt aus elastischem Kunststoff, wie beispielsweise Gummi, gefertigt sind.

An der Überwurfmutter 10 ist ein Vakuumanschluss 13 vorgesehen, durch den das Innere der Kartusche 1 beziehungsweise der Innenraum der Kartusche 1 evakuiert werden kann.

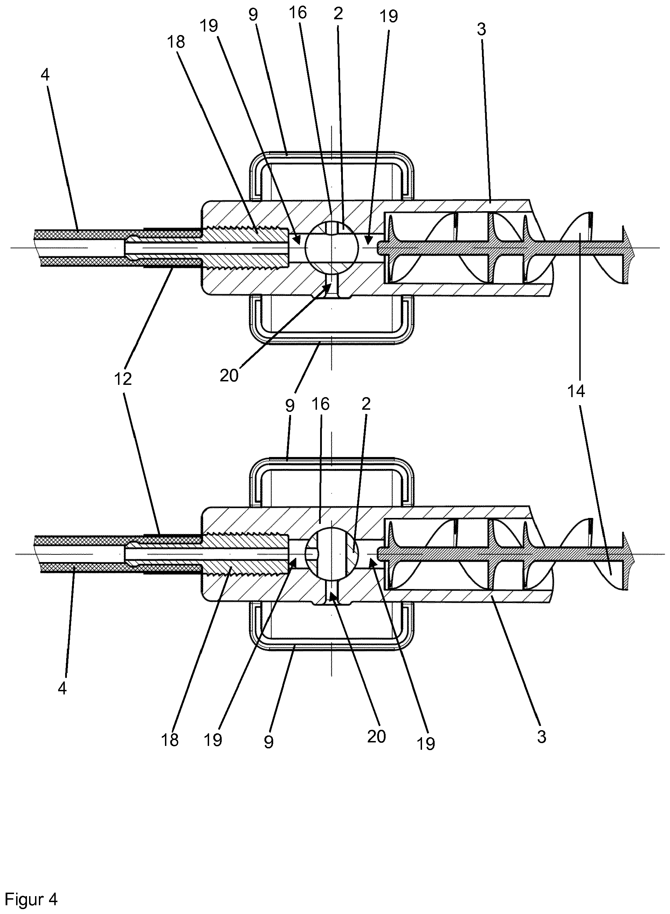

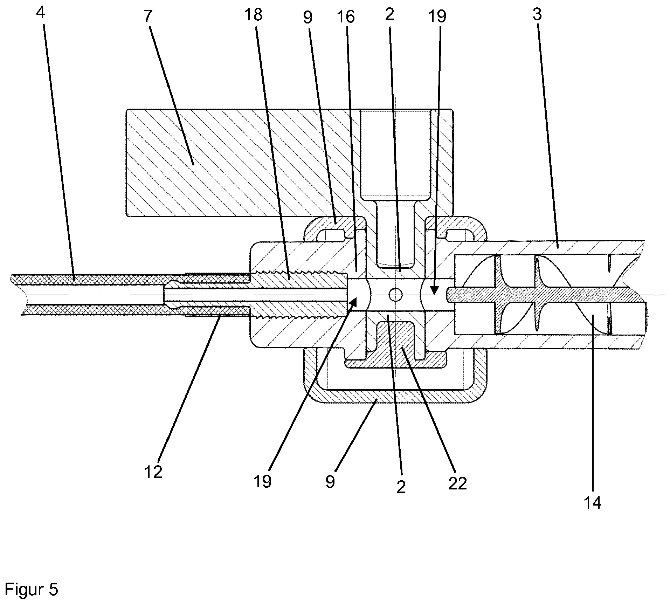

Der für die Erfindung wesentliche Hauptteil des Knochenzement-Applikators ist neben der Kartusche 1 vor allem das Dreiwege-Ventil 2, beziehungsweise insbesondere die Funktionsweise des Dreiwege-Ventils 2 zusammen mit dem Auffangbehälter 9 und mit den im Inneren des Knochenzement-Applikators gebildeten Kanälen. Figur 2 zeigt eine schematische perspektivische Ansicht eines vergrößerten Ausschnitts, in dem das Dreiwege-Ventil 2 des Knochenzement-Applikators nach Figur 1 gezeigt ist. In den Figuren 3 und 4 sind Querschnittansichten durch das Dreiwege-Ventil 2 des Knochenzement-Applikators nach den Figuren 1 und 2 dargestellt und zwar das Dreiwege-Ventil 2 in geschlossener Position beziehungsweise in geschlossener Stellung (Figur 3 oben und Figur 4 unten) und in geöffneter Position beziehungsweise in geöffneter Stellung (Figur 3 unten und Figur 4 oben), um die Funktionsweise des Dreiwege-Ventils 2 anhand des inneren Aufbaus zu erläutern. Zudem ist in Figur 5 eine schematische Querschnittansicht des geöffneten Dreiwege-Ventils nach den Figuren 2, 3 und 4 des Knochenzement-Applikators gezeigt, wobei die Schnittebene senkrecht zu den Schnittebenen nach den Figuren 3 und 4 gewählt ist.

Im Inneren des Rohrs 3 befindet sich ein statischer Mischer 14, der sich bis an das Dreiwege-Ventil 2 heran erstreckt. Mit Hilfe des statischen Mischers 14 werden die Ausgangskomponenten des Knochenzements beziehungsweise der vorgemischte Knochenzementteig durchmischt, wenn diese durch den statischen Mischer 14 im Rohr 3 gepresst werden. Da die Ausgangskomponenten des Knochenzementteigs jedoch bevorzugt zuvor mit einer handgetriebenen Mischeinrichtung im Innenraum der Kartusche 1 durchmischt werden oder ein fertig gemischter Knochenzementteig in die Kartusche 1 eingefüllt wird, ist in dem Rohr 3 bevorzugt kein statischer Mischer 14 angeordnet. Der statische Mischer 14 wird also bevorzugt weggelassen.

Das drehbare Dreiwege-Ventil 2 ist in den Querschnittansichten nach den Figuren 3 und 4 in der Symmetrieebene der darin zu erkennenden Kanäle geschnitten. Die Kanäle sind also zylindrisch und setzen sich in den weggeschnittenen Teil des Dreiwege-Ventils 2 spiegelsymmetrisch fort. Die Kanäle bilden im Dreiwege-Ventil 2 ein T-Stück. Das Dreiwege-Ventil 2 sitzt in einem passenden Ventilsitz 16, der dicht an dem Dreiwege-Ventil 2 anliegt und so die Kanäle abdichtet, wenn diese in dem Ventilsitz 16 gedreht sind. Im Ventilsitz 16 befinden sich zwei Durchgänge 19, durch die der größere, durchgehende Kanal im Dreiwege-Ventil 2 mit dem Rohr 3 auf der einen Seite und mit einem Einsatz 18 aus Metall zum Befestigen des Schlauchs 4 auf der anderen Seite fluiddurchlässig zu verbinden ist.

Senkrecht zu der Achse der beiden Durchgänge 19 befindet sich eine Durchführung 20, die den Ventilsitz 16 mit dem Inneren des nach außen geschlossenen Auffangbehälters 9 verbindet. Der Ventilsitz 16 ist einteilig aus Kunststoff mit dem Rohr 3 ausgeführt. In der geöffneten Position beziehungsweise Stellung des Dreiwege-Ventils 2 (Figur 3 unten, Figur 4 oben und Figur 5) ist der große durchgehende Kanal mit den beiden Durchgängen 19 verbunden und der kleine senkrechte Kanal im Dreiwege-Ventil 2 durch den Ventilsitz 16 geschlossen. Der Knochenzementteig aus der Kartusche 1 kann dann aus dem Rohr 3 durch das Dreiwege-Ventil 2 und den Einsatz 18 in den Schlauch 4 fließen. In der geschlossenen Position beziehungsweise Stellung des Dreiwege-Ventils 2 (Figur 3 oben und Figur 4 unten) ist eine Seite des großen durchgehenden Kanals mit der Durchführung 20 zum Innenraum des Auffangbehälters 9 verbunden und der kleinere senkrechte Kanal mit dem Durchgang 19 zum Schlauch 4 verbunden, während der andere Durchgang 19 zum Rohr 3 durch das Dreiwege-Ventil 2 verschlossen ist. Der Knochenzementteig kann so aus dem Schlauch 4 und gegebenenfalls dem am Luer-System-Adapter 5 angeschlossenen Trokar in den Auffangbehälter 9 fließen. Der Druck hierfür resultiert von einer elastischen Verformung des Schlauchs 4 und gegebenenfalls des Trokars, die sich beim Auspressen beziehungsweise beim Durchpressen des Knochenzementteigs aufgebaut hat.

Das außen zylindrische Dreiwege-Ventil 2 ist durch eine zylindrische Bohrung im Ventilsitz 16 geführt und auf der dem Knebelgriff 7 gegenüberliegenden Seite mit einem Stopfen 22 verbunden und so gegen Herausfallen oder versehentliches Herausziehen aus dem Ventilsitz 16 gesichert.

Durch den erfindungsgemäßen Aufbau gelingt es, dass der Strom des Knochenzementteigs durch Drehen und damit Schließen des Dreiwege-Ventils 2 schnell unterbrochen wird, ohne dass große Mengen des Knochenzementteigs durch die Applikationsöffnung 6 nachströmen. Gleichzeitig werden ein Austreten des Knochenzementteigs und damit eine Kontamination der Umgebung oder des Anwenders mit Hilfe des Auffangbehälters 9 verhindert, der überschüssigen Knochenzementteig aufnimmt. Des Weiteren bleibt der Druck in der Rückseite des Knochenzement-Applikators, also zwischen dem Dreiwege-Ventil 2 und dem Austragskolben 11 in der Kartusche 1, erhalten, so dass nach einem erneuten Öffnen des Dreiwege-Ventils 2 schnell wieder der Fluss des Knochenzementteigs bereitgestellt werden kann, ohne dass der Druck in der Rückseite des Knochenzement-Applikators neu aufgebaut werden muss.

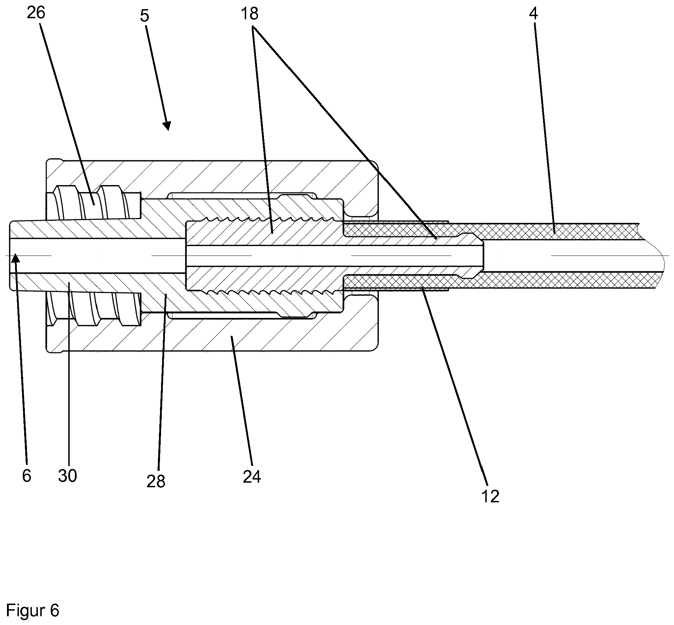

Figur 6 zeigt eine schematische Querschnittansicht des Luer-System-Adapters 5 an der Spitze des Knochenzement-Applikators als Ausschnittvergrößerung. In dem Luer-System-Adapter 5 befindet sich analog zum Anschluss des Ventilsitzes 16 an den Schlauch 4 ein Einsatz 18 aus einem metallischen Werkstoff. Der Schlauch 4 ist mit der Hülse 12 auf diesen Einsatz 18 gecrimpt, um eine druckdichte Verbindung herzustellen. Ansonsten besteht der Luer-System-Adapter 5 aus einer äußeren Hülse 24 mit einem Innengewinde 26 und einem Innenteil 28 mit einem Konus 30. Im Innenteil 28 verläuft ein Kanal, der über einen Kanal des Einsatzes 18 mit dem Schlauch 4 verbunden ist und der auf der anderen Seite in die Applikationsöffnung 6 mündet. Die äußere Hülse 24 und das Innenteil 28 sind aus Kunststoff gefertigt. Theoretisch kann auch ein anderer Adapter vorgesehen sein oder ein Trokar oder ein ähnliches Bauteil kann fest mit dem Schlauch 4 verbunden sein.

Damit der zähe Knochenzementteig mit einer manuell bedienbaren Auspressvorrichtung ausgepresst werden kann, also mit manueller Kraft ausgepresst werden kann, ist der Innendurchmesser der Kartusche 1 nicht zu groß gewählt. Bevorzugt ist der Innendurchmesser kleiner als 35 mm, besonders bevorzugt kleiner als 25 mm. Dadurch wird erreicht, dass der Widerstand, der durch den zähflüssigen Knochenzementteig im Knochenzement-Applikator verursacht wird, nicht so groß wird, dass er mit manueller Kraft nicht mehr ohne weiteres von normalen Anwendern ausgedrückt werden kann.

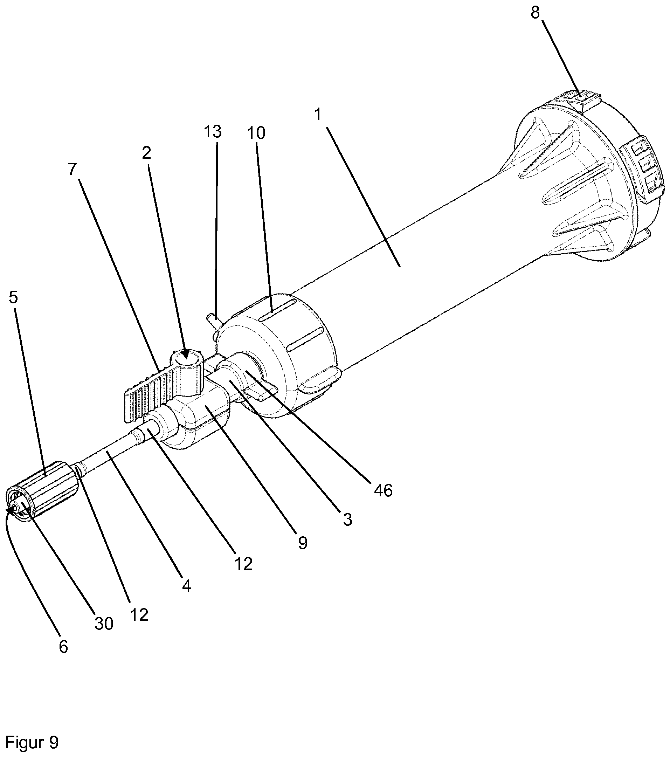

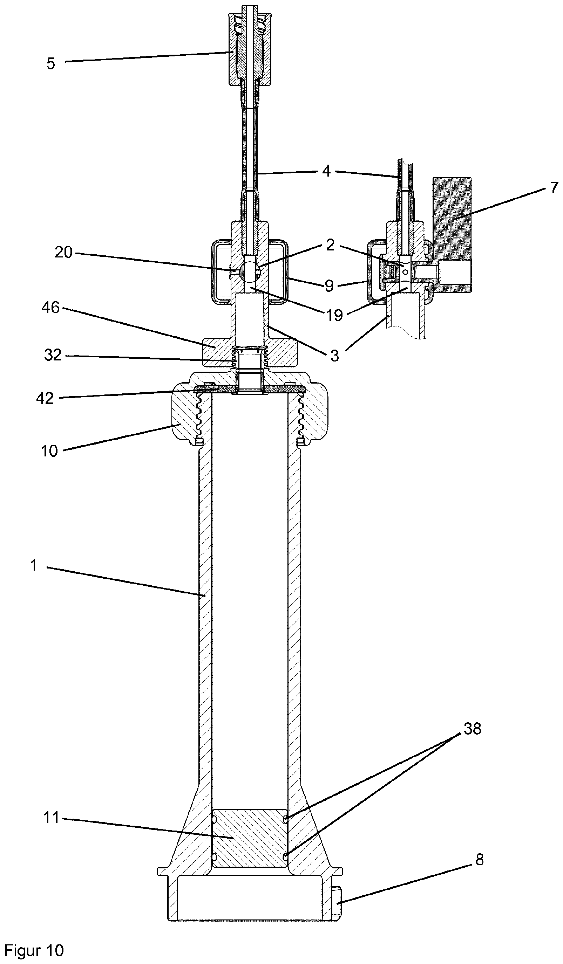

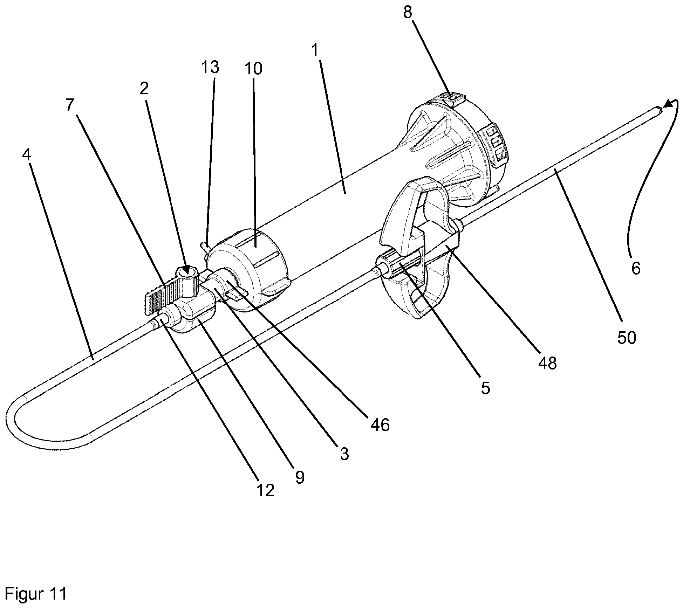

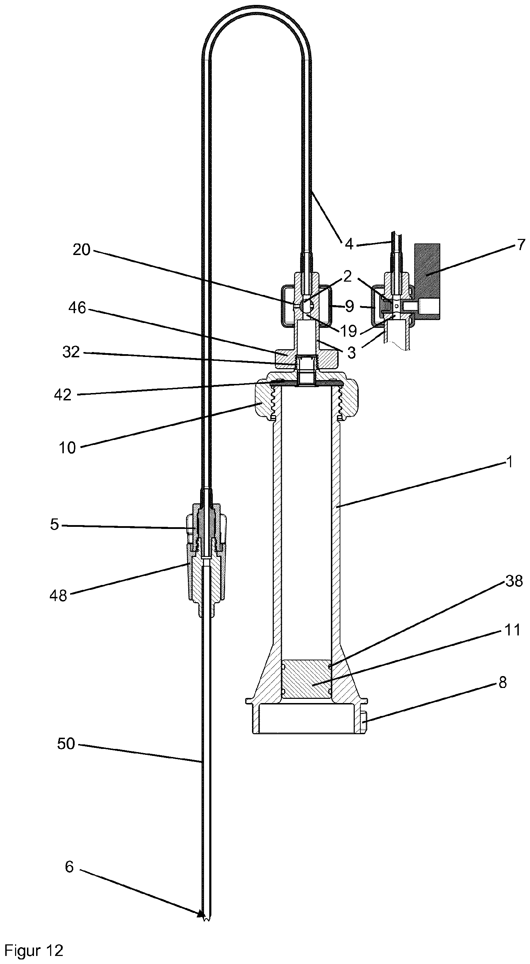

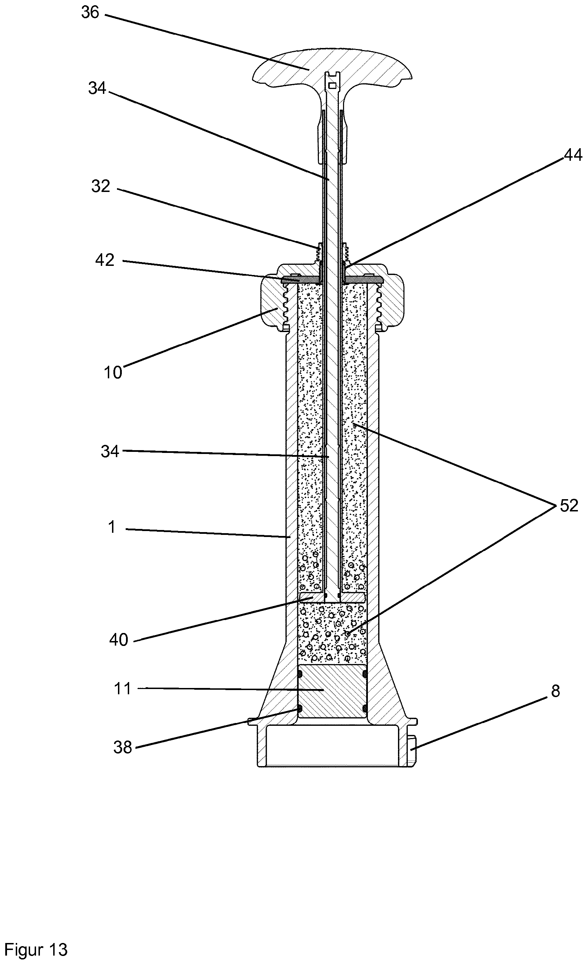

Im Folgenden wird mit den Figuren 7 bis 12 der Knochenzement-Applikator in verschiedenen zusammengesetzten Zuständen gezeigt. Die Figuren 7 und 8 zeigen den Knochenzement-Applikator im Ausgangzustand, die Figuren 9 und 10 mit angesetztem Dreiwege-Ventil und die Figuren 11 und 12 in einem Zustand bereit zur Applikation des Knochenzementteigs. Mit den Figuren 13 bis 17 werden schematische Querschnittansichten des erfindungsgemäßen Knochenzement-Applikators während eines beispielhaften Anwendungsablaufs in chronologischer Reihenfolge gezeigt und so ein Verfahren verdeutlicht.

Im Ausgangszustand (Figuren 7 und 8) ist durch einen Stutzen 32 mit einem Außengewinde, auf den in Figur 1 das Rohr 3 aufgeschraubt ist, ein Mischstab 34 oder ein Mischrohr 34 ins Innere der Kartusche 1 geführt. Der Mischstab 34 kann mit einem Handgriff 36 bedient werden, das heißt gegen die Kartusche 1 gedreht und in Längsrichtung der Kartusche 1 verschoben werden.

Der Austragskolben 11, der an der Rückseite der Kartusche 1 angeordnet ist (in Figur 8 unten), ist mit zwei umlaufenden Dichtungen 38 aus Gummi gegen die Innenwand des zylindrischen Innenraums der Kartusche 1 abgedichtet. An dem ins Innere der Kartusche 1 weisenden Ende des Mischstabs 34 sind mehrere Mischflügel 40 angeordnet, mit denen der Inhalt der Kartusche 1 manuell durchmischt werden kann. Wenn also die Ausgangskomponenten 52 (siehe Figur 13) in die Kartusche eingefüllt sind, können durch Hineinschieben und Herausziehen sowie durch Drehen des Mischstabs 34 die Mischflügel 40 im Inneren der Kartusche 1 bewegt werden und so die Ausgangskomponenten 52 miteinander gemischt werden.

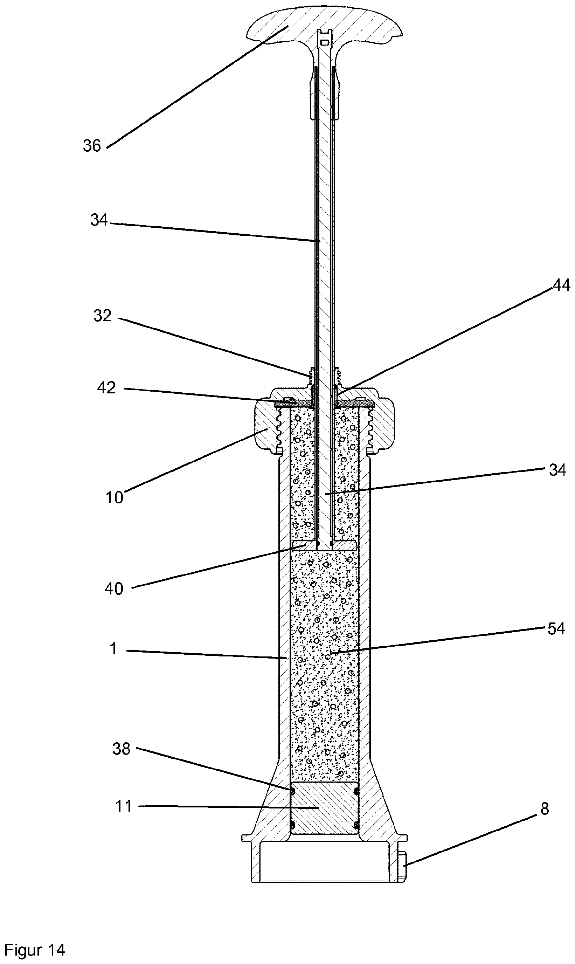

Zwischen der Überwurfmutter 10 und der Vorderkante der Kartuschenwände 1 ist eine Porenscheibe 42 eingeklemmt und dadurch positioniert. Die Porenscheibe 42 ist für Knochenzementpulver nicht durchlässig. Zwischen der Porenscheibe 42 und der Überwurfmutter 10 ist ein ringförmiger oder ringscheibenförmiger Freiraum vorgesehen, der durch eine entsprechende Vertiefung in der Unterseite der Überwurfmutter 10 realisiert ist. Der Vakuumanschluss 13 ist mit diesem Freiraum verbunden. Durch den Vakuumanschluss 13 kann so der Innenraum der Kartusche 1 evakuiert werden, ohne dass Zementpulver aus dem Inneren der Kartusche 1 durch den Vakuumanschluss 13 gelangt, da dieses von der Porenscheibe 42 zurückgehalten wird. Auf diese Weise können die Ausgangskomponenten 52 unter Vakuum miteinander gemischt werden, um einen blasenfreien Knochenzementteig 54 (siehe Figur 14) zu erhalten. Der Mischstab 34 ist durch eine abgedichtete Führung 44 in Form einer Hülse durch die Überwurfmutter 10 geführt, so dass beim Mischen der Ausgangskomponenten 52 diese und auch nicht der Knochenzementteig 54 nicht an dieser Stelle austreten können.

Anschließend wird der Mischstab 34 mit den Mischflügeln 40 aus dem Innenraum der Kartusche 1 herausgezogen. Auf den Stutzen 32 wird nun das Rohr 3 mit dem Dreiwege-Ventil 2 und dem Schlauch 4 mit Hilfe einer Überwurfmutter 46 aufgeschraubt (siehe Figuren 9 und 10). Das Dreiwege-Ventil 2 ist dabei in der geöffneten Stellung beziehungsweise Position dargestellt. Das Dreiwege-Ventil 2 ist in Figur 10 in zwei senkrecht zueinander verlaufenden Längsschnittdarstellungen gezeigt. Die Applikationsöffnung 6 im Luer-System-Adapter 5 steht so in Fluidverbindung mit dem Innenraum der Kartusche 1.

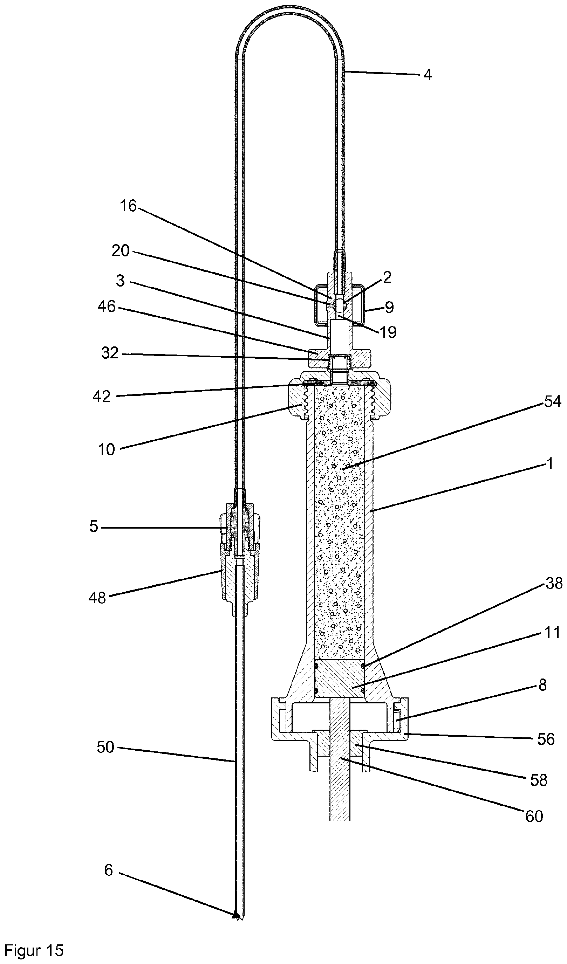

An den Luer-System-Adapter 5 wird nun ein Trokar 48 mit einer Kanüle 50 angeschlossen. Dadurch wird die Applikationsöffnung 6 bis zur Spitze der Kanüle 50 verlängert. Die Applikationsöffnung 6 an der Spitze des Trokars 48 steht nun über das geöffnete Dreiwege-Ventil 2 in Fluidverbindung mit dem Innenraum der Kartusche 1 (siehe Figuren 12 und 15).

Der Knochenzement-Applikator kann zuvor oder nun in eine Auspressvorrichtung eingesetzt werden (siehe Figur 15). Von der Auspressvorrichtung ist in Figur 15 nur ein Anschluss 56, der mit dem Anschluss 8 der Kartusche 1 verbunden ist, eine Lagerung 58 und ein in der Lagerung 58 gelagerter Stößel 60 zu erkennen. Der Stößel 60 kann in Längsrichtung gegen die Lagerung 58 manuell vorgetrieben werden. Der Stößel 60 liegt an der Rückseite des Austragskolbens 11 an, wenn die Auspressvorrichtung mit der Kartusche 1 beziehungsweise mit dem Knochenzement-Applikator verbunden ist.

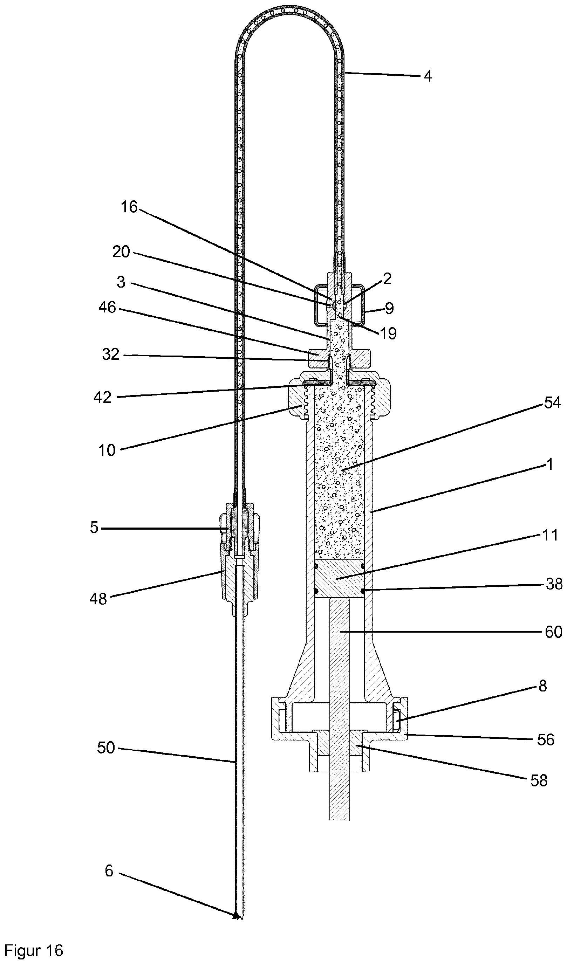

Durch Vortreiben des Austragskolbens 11 mit dem Stößel 60 wird der in der Kartusche 1 enthaltene Knochenzementteig 54 nach vorne durch das Rohr 3 und durch das geöffnete Dreiwege-Ventil 2 in den Schlauch 4 gepresst (siehe Figur 16). Bei weiterem Vortreiben des Austragskolbens 11 wird der Knochenzementteig 54 durch den Trokar 48 ausgetrieben und kann im oder am Wirbel appliziert werden. Der Fluss des Knochenzementteigs 54 kann dabei jederzeit unterbrochen werden, indem das Dreiwege-Ventil 2 geschlossen wird, wie in den Figur 2, Figur 3 oben und Figur 4 unten dargestellt. Der im Schlauch 4 und im Trokar 48 aufgebaute und auf dem Knochenzementteig 54 lastende Druck wird dabei teilweise dadurch abgebaut, dass der Knochenzementteig 48 durch das Dreiwege-Ventil 2 über die Durchführung 20 in den Auffangbehälter 9 fließt. Dadurch wird die Menge des durch die Applikationsöffnung 6 nachströmenden Knochenzementteigs 54 erheblich reduziert. Gleichzeitig bleibt der Druck zwischen dem Dreiwege-Ventil 2 und dem Austragskolben 11 aufrechterhalten. Dadurch ist der Knochenzement-Applikator schnell wieder einsatzbereit, wenn das Dreiwege-Ventil 2 wieder geöffnet wird.

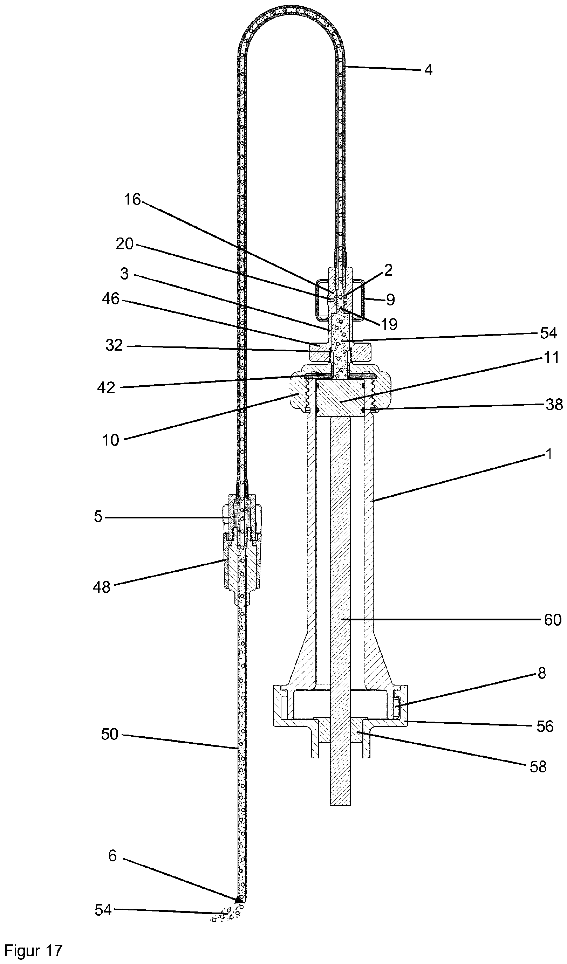

Schließlich ist der Knochenzementteigt 54 durch vollständiges Auspressen aus der Kartusche 1 ausgetragen (siehe Figur 17).

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Kartusche
- 2: Dreiwege-Ventil
- 3: Rohr
- 4: Schlauch
- 5: Luer-System-Adapter
- 6: Applikationsöffnung
- 7: Knebelgriff
- 8: Anschluss
- 9: Auffangbehälter
- 10: Überwurfmutter / Kartuschenkopf
- 11: Austragskolben
- 12: Hülse
- 13: Vakuumanschluss
- 14: Statischer Mischer
- 16: Ventilsitz
- 18: Einsatz
- 19: Durchgang
- 20: Durchführung
- 22: Stopfen
- 24: Äußere Hülse
- 26: Innengewinde
- 28: Innenteil
- 30: Konus
- 32: Stutzen mit Außengewinde
- 34: Mischstab
- 36: Griff
- 38: Dichtung
- 40: Mischflügel
- 42: Porenscheibe
- 44: Dichte Führung
- 46: Überwurfmutter
- 48: Trokar
- 50: Kanüle
- 52: Ausgangskomponenten
- 54: Knochenzementteig
- 56: Anschluss
- 58: Lagerung
- 60: Stößel

## Patentansprüche

1. Knochenzement-Applikator zur Applikation eines Knochenzementteigs (54) im Bereich der Wirbelsäule, der Knochenzement-Applikator aufweisend eine röhrenförmige Kartusche (1) mit einem Innenraum,
einen Austragskolben (11) zum Austreiben der Ausgangskomponenten (52) des Knochenzementteigs (54) oder des Knochenzementteigs (54) aus der Kartusche (1) durch eine dem Austragskolben (11) gegenüberliegende Öffnung der Kartusche (1), wobei der Austragskolben (11) in Längsrichtung im Innenraum der Kartusche (1) beweglich ist,
einen Schlauch (4),
eine Applikationsöffnung (6), durch die der Knochenzementteig (54) zu applizieren ist,
ein von außen bedienbares Dreiwege-Ventil (2), das im Schlauch (4) oder an einer der Kartusche (1) zugewandten Seite des Schlauchs (4) angeordnet oder anzuordnen ist, wobei das Dreiwege-Ventil (2) mit der Öffnung der Kartusche (1) in Fluidverbindung steht, wenn das Dreiwege-Ventil (2) mit der Kartusche (1) verbunden ist,
einen Auffangbehälter (9) zum Aufnehmen von Knochenzementteig (54), der an dem Dreiwege-Ventil (2) angeordnet ist,
wobei das Dreiwege-Ventil (2) derart aufgebaut und in dem Knochenzement-Applikator angeordnet oder anzuordnen ist, dass es
in einer ersten Stellung eine Fluidverbindung zwischen der Applikationsöffnung (6) und der Öffnung der Kartusche (1) bereitstellt und eine Durchführung (20) zum Auffangbehälter (9) verschließt und
in einer zweiten Stellung eine Fluidverbindung zwischen der Applikationsöffnung (6) und dem Auffangbehälter (9) bereitstellt und einen Durchgang (19) zur Öffnung der Kartusche (1) verschließt.

2. Knochenzement-Applikator nach Anspruch 1, **dadurch gekennzeichnet, dass** in Flussrichtung hinter der Öffnung der Kartusche (1) oder zwischen der Öffnung der Kartusche (1) und dem Dreiwege-Ventil (2) oder dem Schlauch (4) ein Mischer (14) zum Mischen des Knochenzementteigs (54), insbesondere ein statischer Mischer (14), angeordnet oder anzuordnen ist, wobei vorzugsweise das Dreiwege-Ventil (2) zwischen dem Mischer (14) und dem Schlauch (4) angeordnet oder anzuordnen ist, so dass das Dreiwege-Ventil (2) in der ersten Stellung eine Fluidverbindung zwischen der Applikationsöffnung (6) und dem Mischer (14) bereitstellt und in der zweiten Stellung den Durchgang (19) zum Mischer (14) verschließt.

3. Knochenzement-Applikator nach Anspruch 1 oder 2, **dadurch gekennzeichnet, dass**
der Knochenzement-Applikator mit Hilfe einer manuell bedienbaren Auspressvorrichtung bedienbar ist und der Austragskolben (11) mit manueller Kraft in der Kartusche (1) bewegbar ist, wobei der Querschnitt des Innenraums der Kartusche (1) maximal 3,5 cm² beträgt, bevorzugt maximal 2,5 cm² beträgt.

4. Knochenzement-Applikator nach einem der vorangehenden Ansprüche, **dadurch gekennzeichnet, dass**
der Schlauch (4) zumindest bereichsweise flexibel ist und/oder die Applikationsöffnung (6) in einem Anschluss (5) mit einem Innengewinde (26), insbesondere in einem Luer-System-Adapter (5), oder in einem Trokar (48) angeordnet ist.

5. Knochenzement-Applikator nach einem der vorangehenden Ansprüche, **dadurch gekennzeichnet, dass**
der Auffangbehälter (9) nach außen für den Knochenzementteig (54) undurchlässig ist, vorzugsweise der Auffangbehälter (9) flüssigkeitsdicht oder flüssigkeitsdicht und gasdicht ist, und/oder der Auffangbehälter (9) ein Volumen aufweist, das mindestens so groß ist wie die Hälfte des Volumens des Schlauchs (4), bevorzugt mindestens so groß ist wie das Volumen des Schlauchs (4).

6. Knochenzement-Applikator nach einem der vorangehenden Ansprüche, **dadurch gekennzeichnet, dass**
die Kartusche (1) an der Rückseite ein Befestigungselement (8) zur Befestigung einer Auspressvorrichtung aufweist.

7. Knochenzement-Applikator nach einem der vorangehenden Ansprüche, **gekennzeichnet durch**
die Kartusche (1) einen zylindrischen Innenraum aufweist und/oder der Austragskolben (11) fluiddicht mit den Innenwänden der Kartusche (1) abschließt, bevorzugt über zumindest eine umlaufende Dichtung (38) und/oder eine Abstreiflippe dicht mit der Innenwand der Kartusche (1) abschließt.

8. Knochenzement-Applikator nach einem der vorangehenden Ansprüche, **dadurch gekennzeichnet, dass**
die Öffnung der Kartusche (1) in einem Kartuschenkopf (10) angeordnet ist, insbesondere in einem mit der Kartusche (1) lösbar verbundenen Kartuschenkopf (10) angeordnet ist, wobei im Bereich der Öffnung ein Befestigungsmittel, insbesondere ein Außengewinde, vorgesehen ist, mit dem der Schlauch (4) oder das Dreiwege-Ventil (2) an der Kartusche (1) zu befestigen ist oder befestigt ist, insbesondere über ein Dichtmittel druckdicht zu befestigen oder befestigt ist.

9. Knochenzement-Applikator nach einem der vorangehenden Ansprüche, **dadurch gekennzeichnet, dass**
eine Mischeinrichtung (34, 36, 40) vorgesehen ist, mit der sich der Inhalt der Kartusche (1) im Innenraum der Kartusche (1) durchmischen lässt, wobei bevorzugt die Mischeinrichtung (34, 36, 40), wenn sie mit der Kartusche (1) verbunden ist, von außen bedienbar ist.

10. Knochenzement-Applikator nach Anspruch 9, **dadurch gekennzeichnet, dass** mehrere mit einem Mischstab (34) verbundene Mischflügel (40) im Innenraum der Kartusche (1) angeordnet sind, die durch eine Bewegung des Mischstabs (34), insbesondere durch axiale lineare Bewegung und durch Drehung des Mischstabs (34), im Innenraum der Kartusche (1) zur Durchmischung von Ausgangskomponenten (52) im Innenraum der Kartusche (1) bewegbar sind, wenn die Mischeinrichtung (34, 36, 40) mit der Kartusche (1) verbunden ist.

11. Knochenzement-Applikator nach einem der Ansprüche 9 oder 10, **dadurch gekennzeichnet, dass**
die Mischeinrichtung (34, 36, 40), insbesondere der Mischstab (34) der Mischeinrichtung (34, 36, 40), durch die Öffnung der Kartusche (1) in den Innenraum der Kartusche (1) geführt ist, wobei die Mischeinrichtung (34, 36, 40) aus der Kartusche (1) entfernbar ist, bevorzugt aus der Kartusche (1) herausziehbar ist, wenn sie mit der Kartusche (1) verbunden ist.

## Claims

1. Bone cement applicator for application of a bone cement dough (54) in the region of the spine, the bone cement applicator comprising
a tubular cartridge (1) with an internal space;
a dispensing plunger (11) for expelling the starting components (52) of the bone cement dough (54) or of the bone cement dough (54) from the cartridge (1) through an opening of the cartridge (1) opposite from the dispensing plunger (11), whereby the dispensing plunger (11) is mobile in longitudinal direction in the internal space of the cartridge (1); a hose (4);
an application opening (6) through which the bone cement dough (54) is applicable;
a three-way valve (2) that is operable from outside and is arranged in the hose (4) or on a side of the hose (4) facing the cartridge (1), whereby the three-way valve (2) is in fluid connection with the opening of the cartridge (2), when the three-way valve (2) is connected to the cartridge (1);
a collecting container (9) arranged on the three-way valve (2) for accommodation of bone cement dough (54);
whereby the three-way valve (2) is appropriately designed and is appropriately arranged or arrangeable in the bone cement applicator such that it,
being in a first position, provides a fluid connection between the application opening (6) and the opening of the cartridge (1) and closes a feed-through (20) to the collecting container (9) and,
being in a second position, provides a fluid connection between the application opening (6) and the collecting container (9) and closes a passage (19) to the opening of the cartridge (1).

2. Bone cement applicator according to claim 1, **characterised in that** a mixer (14) for mixing of the bone cement dough (54), in particular a static mixer (14), is arranged or arrangeable downstream from the opening of the cartridge (1) or between the opening of the cartridge (1) and the three-way valve (2) or the hose (4), whereby the three-way valve (2) preferably is arranged or arrangeable between the mixer (14) and the hose (4), whereby the three-way valve (2), being in the first position, provides a fluid connection between the application opening (6) and the mixer (14) and, being in the second position, closes the passage (19) to the mixer (14).

3. Bone cement applicator according to claim 1 or 2, **characterised in that** the bone cement applicator is operable by means of a manually operated extrusion device and the dispensing plunger (11) is movable in the cartridge (1) by manual force, whereby the cross-section of the internal space of the cartridge (1) is maximally 3.5 cm², preferably is maximally 2.5 cm².

4. Bone cement applicator according to any one of the preceding claims, **characterised in that**
at least part of the hose (4) is flexible and/or the application opening (6) is arranged in a connection (5) with an internal thread (26), in particular in a Luer system adapter (5), or in a trocar.

5. Bone cement applicator according to any one of the preceding claims, **characterised in that**
the collecting container (9) is impermeable for the bone cement dough (54) towards the outside, preferably the collecting container (9) is fluid-tight or fluid-tight and gas-tight, and/or the collecting container (9) has a volume that is at least as large as half the volume of the hose (4), preferably is at least as large as the volume of the hose (4).

6. Bone cement applicator according to any one of the preceding claims, **characterised in that**
the cartridge (1) comprises, on its rear side, an attachment element (8) for attachment of an extrusion device.

7. Bone cement applicator according to any one of the preceding claims, **characterised by**
the cartridge (1) comprises a cylindrical internal space and/or the dispensing plunger (11) closes off in fluid-tight manner against the internal walls of the cartridge (1), preferably closes off tightly against the internal wall of the cartridge (1) by means of at least one circumferential seal (38) and/or a wiper lip.

8. Bone cement applicator according to any one of the preceding claims, **characterised in that**
the opening of the cartridge (1) is arranged in a cartridge head (10), in particular in a cartridge head (10) that is detachably connected to the cartridge (1), whereby an attachment means, in particular an external thread, is provided in the region of the opening by means of which the hose (4) or the three-way valve (2) is connected or connectable to the cartridge (1), in particular is connected or connectable in pressure-tight manner by means of a sealing means.

9. Bone cement applicator according to any one of the preceding claims, **characterised in that**
a mixing facility (34, 36, 40) is provided by means of which the content of the cartridge (1) is mixable in the internal space of the cartridge (1), whereby the mixing facility (34, 36, 40) can preferably be operated from outside when it is connected to the cartridge (1).

10. Bone cement applicator according to claim 9, **characterised in that** multiple mixing vanes (40), connected to a mixing rod (34), are arranged in the internal space of the cartridge (1), and are movable through a motion of the mixing rod (34), in particular through axial linear motion and through rotation of the mixing rod (34), in the internal space of the cartridge (1) in order to mix starting components (52) in the internal space of the cartridge (1), when the mixing facility (34, 36, 40) is connected to the cartridge (1).

11. Bone cement applicator according to any one of the claims 9 or 10, **characterised in that**
the mixing facility (34, 36, 40), in particular the mixing rod (34) of the mixing facility (34, 36, 40), is guided through the opening of the cartridge (1) into the internal space of the cartridge (1), whereby the mixing facility (34, 36, 40) is removable from the cartridge (1), preferably is drawable out of the cartridge (1), when it is connected to the cartridge (1).

## Revendications

1. Applicateur de ciment osseux destiné à une application d'une pâte de ciment osseux (54) dans la zone de la colonne vertébrale, l'applicateur de ciment osseux présentant une cartouche (1) en forme de tube avec un espace intérieur,
un piston d'évacuation (11) pour l'expulsion des composants de départ (52) de la pâte de ciment osseux (54), ou de la pâte de ciment osseux (54), hors de la cartouche (1) par un orifice de la cartouche (1) situé en face du piston d'évacuation (11), le piston d'évacuation (11) étant mobile dans le sens longitudinal dans l'espace intérieur de la cartouche (1),
un tuyau (4),
un orifice d'application (6) par lequel la pâte de ciment osseux (54) est à appliquer, une vanne à trois voies (2), pouvant être actionnée de l'extérieur, qui est disposée ou est à disposer dans le tuyau (4) ou sur le côté du tuyau (4) orienté vers la cartouche (1), la vanne à trois voies (2) étant en communication fluidique avec l'orifice de la cartouche (1) lorsque la vanne à trois voies (2) est reliée avec la cartouche (1),
un réservoir de récupération (9) destiné à la récupération de pâte de ciment osseux (54) qui est disposé sur la vanne à trois voies (2),
la vanne à trois voies (2) étant conçue de telle manière et étant disposée, ou devant être disposée, dans l'applicateur de ciment osseux de sorte qu'elle procure dans un premier réglage une liaison fluidique entre l'orifice d'application (6) et l'orifice de la cartouche (1) et ferme un passage de part en part (20) vers le réservoir de récupération (9), et
procure dans un second réglage une liaison fluidique entre l'orifice d'application (6) et le réservoir de récupération (9) et ferme un passage (19) vers l'orifice de la cartouche (1).

2. Applicateur de ciment osseux selon la revendication 1, **caractérisé en ce que**,
dans le sens d'écoulement, derrière l'orifice de la cartouche (1) ou entre l'orifice de la cartouche (1) et la vanne à trois voies (2) ou le tuyau (4), un mélangeur (14) est disposé, ou est à disposer, pour le mélange de la pâte de ciment osseux (54), en particulier, un mélangeur (14) statique, la vanne à trois voies (2) étant disposée ou devant être disposée, de préférence, entre le mélangeur (14) et le tuyau (14) de sorte que la vanne à trois voies (2) procure une liaison fluidique entre l'orifice d'application (6) et le mélangeur (14) dans le premier réglage et ferme le passage (19) vers le mélangeur (14) dans le second réglage.

3. Applicateur de ciment osseux selon la revendication 1 ou la revendication 2, **caractérisé en ce que**
l'applicateur de ciment osseux peut être actionné à l'aide d'un dispositif d'expulsion actionné manuellement et le piston d'évacuation (11) peut être déplacé dans la cartouche (1) avec une force manuelle, la section transversale de l'espace intérieur de la cartouche (1) étant au maximum de 3,5 cm², de préférence, étant au maximum de 2,5 cm².

4. Applicateur de ciment osseux selon l'une des revendications précédentes, **caractérisé en ce que**
le tuyau (4) est souple au moins par endroits et/ou l'orifice d'application (6) est disposé dans un raccord (5) avec un filetage intérieur (26), notamment, dans un adaptateur de système Luer (5) ou dans un trocart (48).

5. Applicateur de ciment osseux selon l'une des revendications précédentes, **caractérisé en ce que**
le réservoir de récupération (9) est imperméable vers l'extérieur pour la pâte de ciment osseux (54), de préférence, le réservoir de récupération (9) est étanche vis-à-vis des liquides, ou étanche vis-à-vis des liquides et des gaz, et/ou le réservoir de récupération (9) présente un volume qui est au moins aussi grand que la moitié du volume du tuyau (4), de préférence, au moins aussi grand que le volume du tuyau (4).

6. Applicateur de ciment osseux selon l'une des revendications précédentes, **caractérisé en ce que**
la cartouche (1) présente un élément de fixation (8) sur la face arrière pour la fixation d'un dispositif d'expulsion.

7. Applicateur de ciment osseux selon l'une des revendications précédentes, **caractérisé en ce que**
la cartouche (1) présente un espace intérieur cylindrique et/ou le piston d'évacuation (11) se ferme avec les parois intérieures de la cartouche (1) de manière étanche aux fluides, de préférence, par le biais d'un joint (38) périphérique et/ou une lèvre d'essuyage se ferme de manière étanche avec la paroi intérieure de la cartouche (1).

8. Applicateur de ciment osseux selon l'une des revendications précédentes, **caractérisé en ce que**
l'orifice de la cartouche (1) est disposé dans une tête de cartouche (10), notamment est disposé dans une tête de cartouche (10) reliée de manière amovible avec la cartouche (1), où un système de fixation, notamment un filetage extérieur, est prévu dans la zone de l'orifice, avec lequel le tuyau (4), ou la vanne à trois voies (2), est fixé(e) ou est à fixer sur la cartouche (1), en particulier est à fixer ou est fixé(e) de manière étanche en pression par le biais d'un système d'étanchéité.

9. Applicateur de ciment osseux selon l'une des revendications précédentes, **caractérisé en ce**
**qu'**un dispositif de mélange (34, 36, 40) est prévu avec lequel le contenu de la cartouche (1) se mélange dans l'espace intérieur de la cartouche (1), où, de préférence, le dispositif de mélange (34, 36, 40), lorsqu'il est relié avec la cartouche (1), peut être actionné de l'extérieur.

10. Applicateur de ciment osseux selon la revendication 9, **caractérisé en ce que** plusieurs ailerons de mélange (40) reliés avec une tige de mélange (34) sont disposés dans l'espace intérieur de la cartouche (1), qui peuvent être mis en mouvement dans l'espace intérieur de la cartouche (1) par un déplacement de la tige de mélange (34), en particulier, par un déplacement linéaire axial et par une rotation de la tige de mélange (34) dans l'espace intérieur de la cartouche (1) pour un mélange complet des composants de départ (52) lorsque le dispositif de mélange (34, 36, 40) est relié avec la cartouche (1).

11. Applicateur de ciment osseux selon l'une des revendications 9 ou 10, **caractérisé en ce que**
le dispositif de mélange (34, 36, 40), notamment une tige de mélange (34) du dispositif de mélange (34, 36, 40), est mené(e) à travers l'orifice de la cartouche (1) dans l'espace intérieur de la cartouche (1), le dispositif de mélange (34, 36, 40) pouvant être retiré de la cartouche (1), de préférence, pouvant être tiré hors de la cartouche (1), lorsqu'il est relié avec la cartouche (1).
